# EUROPEAN PATENT APPLICATION

(11) **EP 1 839 609 A1**
(43) Date of publication of application: **03.10.2007**
(21) Application number: 07251321.1
(22) Date of filing: 28.03.2007
(51) Int. Cl.: A61B 17/74, A61B 17/78

(54) **Variable angle intramedullary nail assembly**

(30) Priority: 31.03.2006 US 395404
(71) Applicant: DePuy Products, Inc., Warsaw, IN 46581 (US)
(72) Inventor: Metzinger, Anthony J, Winona Lake, IN 46590 (US)
(74) Representative: Belcher, Simon James

(57) **Abstract**

An intramedullary nail assembly for use in a medullary canal of a long bone includes includes a body for positioning at least partially in the medullary canal. The body defines a body aperture through the body and a longitudinal axis of the body and an orientation feature. The orientation feature is connected to the body. The orientation feature is adapted to support the bushing so that the position of the bushing with respect to the body can be adjusted, so that the bushing may receive the screw in a plurality of angular orientations with respect to the longitudinal axis of the body. The angular orientations define a plurality of non-coincident planes.

## Description

The skeletal system includes many long bones that extend from the human torso. These long bones include the femur, fibula, tibia, humerus, radius and ulna. These long bones are particularly exposed to trauma from accidents, and as such often are fractured during such trauma and may be subject to complex devastating fractures.

Automobile accidents, for instance, are a common cause of trauma to long bones. In particular, the femur and tibia frequently fracture when the area around the knee is subjected to a frontal automobile accident.

Often the distal end or proximal portions of the long bone, for example the femur and the tibia, are fractured into several components and must be realigned. Mechanical devices, commonly in the forms of pins, plates, screws, nails, wires and external devices are commonly used to attach fractured long bones. The pins, plates, wires, nails and screws are typically made of a durable material compatible to the human body, for example titanium, stainless steel or cobalt chromium.

Fractures of the long bone are typically secured into position by at least one of three possible techniques.

The first method is the use of intramedullary nails that are positioned in the intramedullary canal of those portions of the fractured bone.

A second method of repairing fractured bones is the use of internal bone plates that are positioned under the soft tissue and on the exterior of the bone and bridges the fractured portion of the bone.

Another method of securing fractured bones in position is the use of external fixators. These external fixators have at least two general categories. In one category the fixator is generally linear with a first portion of the fixator to connect to a first fracture segment of the bone and a second fracture segment of the fixator to connect to the second fracture segment of the bone. A first series of bone screws or pins are first connected to the fixator and then into the first portion of the bone. Then a second series of screws or pins are connected to the fixator and then to the second fracture segment of the bone, thereby securing the first portion fracture segment of the bone to the second portion of the bone.

A second method of external fixation is through the use of a ring type fixator that uses a series of spaced-apart rings to secure the bone. For example, an upper ring and a lower ring are spaced apart by rods. A plurality of wires is placed through the long bone and is connected on each end of the long bone by the ring. The wires are then tensioned much as a spoke in a bicycle are tightened, thereby providing for a rigid structure to support the first fracture segment portion of the bone. Similarly, a plurality of wires are positioned through the second fracture segment of the bone and are secured to and tensioned by the lower ring to provide a rigid fixation of the second fracture segment of the bone bridging the fracture site.

There are a variety of devices used to treat femoral fractures. Fractures of the neck, head or intertrochanter of the femur have been successfully treated with a variety of compression screw assemblies, which include generally a compression plate having a barrel member, a lag screw and a compressing screw. The compression plate is secured to the exterior of the femur and the barrel member is inserted into a predrilled hole in the direction of the femoral head.

The lag screw which has a threaded end and a smooth portion is inserted through the barrel member so that it extends across the break and into the femoral head. The threaded portion engages the femoral head. The compressing screw connects the lag screw to the plate. By adjusting the tension of the compressing screw the compression (reduction) of the fracture can be adjusted. The smooth portion of the lag screw must be free to slide through the barrel member to permit the adjustment of the compression screw.

Subtrochanteric and femoral shaft fractures have been treated with the help of intramedullary rods, which are inserted into the marrow canal of the femur to immobilize the femur parts involved in fractures. A single angled cross-nail or locking screw is inserted through the femur and the proximal end of the intramedullary rod. In some varieties, one or two screws may also be inserted through the femoral shaft and through the distal end of the intramedullary rod. The standard intramedullary rods have been successfully employed in treating fractures in lower portions of the femoral shaft.

Trochanteric nails for use in preparing femoral neck fractures utilize a screw in the form of, for example, a lag screw.

The proximal femoral fractures, for example, those around the less trochanter, greater trochanter, and femoral neck have been successful treated with a variety of compression screw assemblies and intramedullary rods. The intramedullary rods are inserted into the narrow canal of the femur to immobilize the femur parts involved in the fracture. Typically, a single screw is inserted through the femur and the proximal end of the intramedullary rod. Alternatively, a second screw may be inserted through the femur and into the proximal end of the intramedullary rod to prevent rotation of, for example, the neck and head of the femur.

Intramedullary rods or nails are often used in the femur to repair shaft fractures or neck fractures of the femur. The intramedullary canal of the femur and the centerline of the neck form an angle between each other. The angle between the femur and the neck of the femur may vary from patient to patient. Attempts have been made to accommodate the variation in the neck to shaft angle of the femur of patients. For example, intramedullary nails have been provided that provide for differing femoral neck angles. For example, a femoral neck angle of 125°, 130° and 135° have been offered. This solution is not optimal because if the surgeon would desire to change this angle from the offered angles, such nails are not available. Also, this solution requires the inventory of three different intramedullary nails each with its own femoral neck angle. Further, the femoral neck may have a fracture with a fracture pattern that may align with the femoral neck angle of the prosthesis. For such fractures in a patient, it may be desirable to provide a femoral neck angle that provides an angle different than that of the fracture pattern so that the neck may be properly repaired.

The present invention is directed to alleviate at some of the aforementioned concerns with orthopaedic fasteners.

According to the present invention, an intramedullary nail with a rotating sphere placed approximately along the longitudinal axis of the nail is provided. The sphere is allowed to pivot about its centre with the support of opposed concave cradles. The cradles support the sphere but are not fixed to the sphere, which allows the sphere to pivot. The sphere can be positioned at the desired angle and locked into position with a locking device through, for example, the centre of the nail.

The invention may be in the form of an intramedullary nail, for example, a femoral nail, a tibial nail or any nail that may be fitted into the canal of a long bone. The nail includes a pivoting ball or sphere in the body of the nail. The pivoting sphere or ball allows a screw to be positioned through the nail at various angles. The screw may be placed for example, normal to the central axis of the nail or at angles up to but not limited to 45° from the normal direction. The screws may also be placed in a variety of planes that intersect the central axis. The sphere may then be locked with a locking device. For example, the locking device may be in the form of a locking plug with external threads mated with internal threads in the nail to secure the barrel at the selected, optimum angle.

According to one embodiment of the present invention, there is provided an intramedullary nail assembly for use in a medullary canal of a long bone. The assembly includes a nail for positioning at least partially in the medullary canal. The nail defines an aperture through the nail. The nail further defines a longitudinal axis of the nail. The assembly also includes a bushing and a screw. The bushing may be positioned at least partially in the aperture and adapted to receive the screw in a plurality of angular orientations with respect to the longitudinal axis of the nail. The angular orientations define a plurality of non-coincident planes.

According to another embodiment of the present invention, there is provided an intramedullary nail kit for use in a medullary canal of a long bone. The kit includes a nail for positioning at least partially in the medullary canal. The nail has a first internal wall defining a nail opening through the wall. The nail further defines a longitudinal axis of the nail. The kit also includes a screw for cooperation with the opening of the nail and a bushing. The bushing can be fitted at least partially in the aperture and adapted to receive the screw in a plurality of angular orientations with respect to the longitudinal axis of the nail. The angular orientations define a plurality of non-coincident planes. The kit also includes a device for positioning at least one of the screw and the bushing with respect to the nail.

The devices provided by the invention can be used in a method for performing trauma surgery on a long bone. The method includes the step of providing an intramedullary nail. The nail defines an aperture through the nail. The aperture has a centerline of the aperture. The centerline of the aperture is adjustable in a plurality of non-coincident planes. The method also includes the steps of positioning the nail at least partially in the medullary canal and providing a screw for attachment to the long bone. The method also includes the steps of attaching the screw to the nail and moving the aperture centerline with respect to the nail to form an angle between the nail longitudinal axis and the aperture longitudinal axis.

According to yet another embodiment of the present invention, there is provided an intramedullary nail assembly for use in a medullary canal of a long bone. The assembly includes a body for positioning at least partially in the medullary canal. The body defines a body aperture through the body. The body further defines a longitudinal axis of the body and an orientation feature. The orientation feature is connected to the body. The orientation feature is adapted to support the bushing so that the position of the bushing with respect to the body can be adjusted so that the bushing may receive the screw in a plurality of angular orientations with respect to the longitudinal axis of the body. The angular orientations define a plurality of non-coincident planes.

According to yet another embodiment of the present invention, there is provided an intramedullary nail assembly for use with a screw in a medullary canal of a long bone. The assembly includes a nail for positioning at least partially in the medullary canal. The nail has a first internal wall defining a nail opening through the wall. The nail further defines a longitudinal axis of the nail and a bushing rotatably positioned at least partially in the nail opening. The bushing is adapted to receive the screw in a plurality of angular orientations with respect to the longitudinal axis of the nail. The plurality of angular orientations defines a plurality of non-coincident planes.

The devices provided by the invention can be used in a method for performing trauma surgery on a long bone. The method includes the steps of providing a screw for attachment to the long bone and providing an intramedullary nail. The nail defines an aperture through the nail. The aperture closely conforms to the screw. The orientation of the centerline of the aperture with respect to the nail can be varied and locked. The nail is provided with the centerline being locked in a preselected one of the variable centerline orientations. The variable centerlines define a plurality of non-concurrent planes. The method includes the steps of implanting the nail at least partially in the medullary canal and attaching a screw through the aperture and into the long bone.

According to another embodiment of the present invention, there is provided a fixture for use with an intramedullary nail having nail body and a screw feature for receiving a screw whose orientation with respect to the nail body can be adjusted. The fixture is adapted to orient the screw feature with respect to the nail. The fixture includes a first portion for cooperation with the nail body and a second portion for cooperation with the nail body.

The fixture also includes a third portion for cooperation with the screw feature. The third portion is capable of being positioned in a plurality of positions with respect to the first portion. The fixture also includes a first nail body-positioning feature for positioning the nail body with respect to the first portion of the fixture. The fixture also includes a second nail body-positioning feature for positioning the nail body with respect to the second portion of the fixture. The fixture also includes a screw feature-positioning feature for positioning the screw feature with respect to the third portion of the fixture.

According to another embodiment of the present invention, there is provided a kit for use in performing arthroplasty. The kit includes a nail including a nail body for positioning at least partially in the medullary canal. The nail body has a first internal wall defining a nail opening through the nail. The nail body further defines a longitudinal axis of the nail. The nail further includes a screw feature positioned at least partially in the nail opening and defining an opening in the screw feature. The opening defines a longitudinal axis of the opening. The screw feature is adapted for movement to orient the longitudinal axis of the opening in a plurality of angular positions with respect to the longitudinal axis of the nail body such that the plurality of positions of the longitudinal axis of the opening define a plurality of non-coincident planes.

The kit also includes a screw which can be fitted at least partially within the opening of said screw feature and a fixture. The fixture includes a nail body portion for cooperation with the nail body and a screw feature portion for cooperation with the screw feature. The screw feature portion is capable of being positioned in a plurality of positions with respect to the nail body portion. The fixture also includes a nail positioning feature for positioning the nail with respect to the nail body portion of the fixture, and a screw feature-positioning feature for positioning the screw feature with respect to the screw feature portion of the fixture.

The devices provided by the invention can be used in a method for performing trauma surgery on a long bone of a patient. The method includes the steps of providing an intramedullary nail assembly including a nail body and a screw feature. The screw feature defines an opening defining an opening centerline which can be positioned in a range of orientations with respect to the nail body. The plurality of orientations of the opening centerline define a plurality of non-coincident planes.

The method also includes the steps of cutting an incision on the patient to expose the long bone and obtaining patient specific data related to the shape of one of the patient's bones. The method also includes the steps of determining the proper angular relationship of the screw feature with respect to the nail body based on the patient specific data and providing a fixture for setting the angular position of the screw feature with respect to the nail body. The method also includes the steps of setting the angular position of the screw feature with respect to the nail body at the proper angular relationship with the fixture and implanting the nail assembly into the patient.

The technical advantages of the present invention include the ability to place a screw at various angles with respect to the longitudinal axis of an intramedullary nail. The placement of the screw at a varying angle can accommodate the variation from patient to patient in the neck shaft angle of, for example, the femur or to position the screw at a proper angular position with respect to the fracture that the screw is to bridge.

For example, according to one aspect of the present invention, an intramedullary nail for use with a screw in a medullary canal of a long bone is provided. The assembly includes a nail for positioning in the canal. The canal includes an aperture through the nail. The nail further defines a longitudinal axis. The nail assembly further includes a bushing position in the aperture and adapted to receive the screw in a plurality of angular positions.

Thus, the present invention provides for the ability to place a screw at varying angles with respect to the nail. Thus, the present invention provides the ability to provide a screw at varying angles with respect to the nail. The varying angles may accommodate variations in anatomy and variations in the position of the fracture, particularly the fracture of a neck.

The technical advantages of the present invention further include the ability to lock the pivoting barrel at any one of various angles, thereby providing for a predetermined fixed angle for a screw, particularly for a femoral neck screw for a femoral intramedullary nail.

For example, according to another aspect of the present invention, an intramedullary nail for use with a screw in a medullary canal is provided. The nail is positioned at least partially in the nail and includes an aperture. A bushing is positioned in the aperture and is adapted to receive the screw in a plurality of angular orientations. A locking device is associated with the nail for locking the bushing in a fixed particular angle. Thus, the present invention provides for the ability to lock the pivoting spherical bushing at a predetermined selected angle.

The technical advantages of the present invention further include the ability to accommodate the variations in human anatomy and variations in fracture locations by providing an intramedullary nail assembly with a screw at a specific angle relative to the nail. For example, according to yet another aspect of the present invention, an intramedullary nail for use with a screw in a medullary canal is provided.

The nail assembly includes a nail positioned partially in the canal and defining an opening through the nail. The assembly also includes a bushing positioned in the aperture and adapted to receive the screw at a predetermined angle with respect to the longitudinal axis of the nail. Thus, the present invention provides for an intramedullary nail having a screw that accommodates variations in the human anatomy and fracture locations by providing a screw at a specific angle with respect to the longitudinal axis of the nail.

The technical advantages of the present invention include the ability to reduce inventory of intramedullary nails at a hospital or at a manufacturer's facility. For example, according to yet another aspect of the present invention, an intramedullary nail assembly is provided including a nail defining an aperture and a bushing fitted in the nail and adapted to be positioned in a plurality of positions. The nail assembly further includes a screw that may be positioned in the bushing to provide for a nail assembly with a plurality of angular relationships with respect to the nail.

Thus, the present invention provides for an intramedullary nail that includes a screw that may be positioned at various angular positions. By providing the nail assembly with a screw that may be positioned at various angular positions, an individual nail assembly is not necessary for each particular angular position or range of angular positions, thereby reducing inventory. Thus, the present invention provides for reduced inventory of nail assemblies.

The technical advantages of the present invention include the ability to provide a femoral intramedullary nail with a screw that may be positioned in the ideal angular position in the neck of the femur. For example, according to another aspect of the present invention, an intramedullary nail assembly is provided with a nail, including an opening and a bushing fitted in the nail that is rotatably positioned with respect to the nail. The nail assembly further includes a screw that is fitted into an opening in the bushing. The screw may be rotatably positioned with respect to the nail to position the nail in the ideal position in the patient. Thus, the present invention provides for an intramedullary nail, which may position a screw in the optimal position in the neck of the femur.

The technical advantages of the present invention also include the ability to provide an intramedullary nail with a screw that may be positioned at the ideal angle between the greater trochanter and the lesser trochanter. For example, according to yet another aspect of the present invention, an intramedullary nail assembly is provided including a nail having an aperture and a bushing whose rotational orientation in the opening can be adjusted. The bushing includes an opening for receiving a screw to be positioned at an angle to extend from the greater trochanter to the lesser trochanter. Thus, the present invention provides for an intramedullary nail that works with a screw that may be positioned in the ideal angular position with respect to the greater trochanter and lesser trochanter.

The technical advantages of the present invention further include the ability to accommodate fractures in the neck of the femur and fractures related to the greater trochanter and lesser trochanter with the same nail.

For example, according to yet another aspect of the present invention, an intramedullary nail assembly is provided, including a nail having an opening in the nail. The opening of the nail receives a bushing, whose rotational orientation within the nail can be adjusted. The bushing includes an opening for receiving a screw, which may be rotatably positioned from a first position, in which the screw is in alignment with the neck of the femur, and a second position in which the screw is positioned with respect to the greater trochanter and lesser trochanter. Thus, the present invention provides for an intramedullary nail assembly that may be used for both greater and lesser trochanter fractures and for femoral neck fractures.

The technical advantages of the present invention further include the ability to provide for an intramedullary nail that may be preset to the specific requirements of a patient. For example, according to yet another aspect of the present invention, an intramedullary nail kit is provided. The kit includes a nail having an opening for receiving a bushing and a bushing rotatably fitted in the opening. The bushing includes an opening for receiving a screw. The nail assembly further includes a locking mechanism for locking the bushing with respect to the nail in a particular angular relationship.

The kit further includes an alignment device for presetting or aligning the position of the bushing with respect to the nail and permitting the aligned position of the bushing with respect to the nail to be locked in place with the locking mechanism. Thus, the present invention provides for an intramedullary nail assembly that may be preset to a given position.

The technical advantages of the present invention further include the ability to allow two separate screws to be placed at one time in one of two different planes. For example, and according to another aspect of the present invention, an intramedullary nail for use in a medullary canal of a long bone is provided. The assembly includes a nail for positioning at least partially in the medullary canal. The nail defines an aperture through the nail. The nail further defines a longitudinal axis of the nail. The assembly also includes two bushings and two screws. Each bushing is adapted to be positioned at least partially in an aperture and adapted to receive one of the screws in a plurality of angular orientations with respect to the longitudinal axis of the nail. The plurality of angular orientations define a plurality of non-coincident planes. Thus, the present invention provides for the ability to allow two separate screws, one in each of two openings to be placed at one time in or two different planes.

The technical advantages of the present invention also include the ability to allow for multiple screw fixations to be achieved in opposing planes for better fracture stabilization. For example, according to yet another aspect of the present invention, an intramedullary nail for use in the canal of a long bone is provided. The nail assembly includes a nail for positioning at least partially in the medullary canal. The nail defines aperture through the nail. The nail further defines a longitudinal axis of the nail. The nail assembly further includes a bushing and a screw. The bushing is adapted to be positioned at least partially in the aperture and adapted to receive the screw in a plurality of angular orientations with respect to the longitudinal axis of the nail. The plurality of angular orientations defines a plurality of non-coincident planes.

The technical advantages of the present invention also include the ability to place two screws in the same plane of the femoral neck. For example, according to yet another aspect of the present invention, an intramedullary nail assembly is provided for use in the medullary canal of a long bone. The assembly includes a nail for positioning at least partially in the medullary canal. The nail defines an aperture through the nail. The nail further defines a longitudinal axis of the nail. The assembly also includes a bushing and a screw. The bushing is adapted to be positioned at least partially in the aperture. The nail assembly further includes a second bushing defining a second bushing opening for receiving at least a portion of the screw. The second bushing is adapted to be positioned at least partially in the second bushing opening and adapted to receive the second screw in a plurality of angular orientations with respect to the longitudinal axis of the nail. Thus, the present invention provides for two screws in the same plane of the femoral neck.

The technical advantages of the present invention also include the ability to place screws in multiple planes to treat unstable femoral fractures. For example, according to yet another aspect of the present invention, an intramedullary nail assembly is provided for use in the medullary canal of a long bone. The assembly includes a nail for positioning at least partially in the canal. The nail defines an aperture and further defines a longitudinal axis. The nail assembly includes a bushing and a screw. The bushing is adapted to be positioned at least partially in the aperture and adapted to receive the screw in a plurality of angular orientations with respect to the longitudinal axis of the nail. The plurality of angular orientations define a plurality of non-coincident planes. Thus, the present invention provides for a nail in which screws may be placed in multiple planes to treat unstable femoral fractures.

Embodiments of the invention will now be described by way of example with reference to the accompanying drawings, in which:
FIG. 1 is a partial anterior/posterior view of an intramedullary nail assembly with a compression screw for use with a piriformis entry in accordance with an embodiment of the present invention;
FIG. 1A is a partial top view of the nail assembly of FIG. 1;
FIG. 2 is an partial medial/lateral view of the intramedullary nail assembly of FIG. 1;
FIG. 3 is an enlarged partial medial/lateral view of the intramedullary nail of FIG. 2;
FIG. 4 is an partial anterior/posterior view of the intramedullary nail assembly of FIG. 1;
FIG. 4A is a partial medial/lateral view of FIG. 4;
FIG. 5 is an enlarged partial medial/lateral view of the aperture of the intramedullary nail of the nail assembly of FIG. 2;
FIG. 5A is a partial anterior/posterior view of the aperture of the nail of FIG. 5;
FIG. 5B is a partial anterior/posterior view of an aperture with chamfers for additional angular movement according to another embodiment of the present invention;
FIG. 6 is an enlarged partial medial/lateral view of the proximal portion of the intramedullary nail of the nail assembly of FIG. 2;
FIG. 7 is an enlarged partial perspective view of the proximal portion of the intramedullary nail of the nail assembly of FIG. 2;
FIG. 8 is a partial anterior/posterior view of the proximal portion of the intramedullary nail assembly of FIG. 2;
FIG. 9 is a plan view partially in cross section of the locking plug of the intramedullary nail assembly of FIG. 2;
FIG. 10 is a partial plan view partially in cross section of the nail of the intramedullary nail assembly of FIG. 2 showing the threaded opening for receiving the locking plug of FIG. 10;
FIG. 11 is a plan view of the bushing of the intramedullary nail assembly of FIG. 2;
FIG. 12 is an end view of the bushing of FIG. 11;
FIG. 13 is an anterior/posterior view of the nail assembly of FIG. 2;
FIG. 14 is a medial/lateral view of the nail assembly of FIG. 13 showing the curvature of the middle portion of the nail;
FIG. 15 is a partial medial/lateral view of the distal portion of the nail assembly of FIG. 14;
FIG. 16 is a partial medial/lateral view partially in cross section of another embodiment of the present invention in the form of an intramedullary nail with four distal apertures;
FIG. 17 is an anterior/posterior view of the intramedullary nail assembly of FIG. 1 showing the screw in an oblique position;
FIG. 17A is an anterior/posterior view of another embodiment of the present invention in the form of a nail assembly with a distal adjustable bushing;
FIG. 18 is a plan view of a cortical screw for use in the distal openings of the intramedullary nail assembly of FIG. 1;
FIG. 19 is a plan view of a partially threaded cancellous lag screw for use in the oblique proximal opening of the intramedullary nail assembly of FIG. 1;
FIG. 19A is a plan view of a fully threaded cancellous lag screw for use in the oblique proximal opening of the intramedullary nail assembly of FIG. 1;
FIG. 20 is a partial enlarged cross-sectional view of the box-shaped thread of the lag screw of FIG. 19;
FIG. 20A is a partial enlarged cross-sectional view of a standard screw thread shaped thread that may be an alternative construction of the lag screw of the present invention;
FIG. 20B is a partial view of a V-shaped thread that may be an alternative construction of the lag screw for use with an intramedullary nail assembly of the present invention;
FIG. 20C is a partial view of a rectangular-shaped thread that may be an alternative construction of the lag screw for use with an intramedullary nail assembly of the present invention;
FIG. 20D is a partial view of an a truncated V-shaped thread that may be an alternative construction of the lag screw for use with an intramedullary nail assembly of the present invention;
FIG. 20E is a partial view of a reverse box-shaped thread that may be an alternative construction of the lag screw for use with an intramedullary nail assembly of the present invention;
FIG. 20F is a partial view of a simple box-shaped thread that may be an alternative construction of the lag screw for use with an intramedullary nail assembly of the present invention;
FIG. 21 is a partial anterior/posterior view of the nail implant assembly of FIG. 1 showing the screw in a plurality of positions;
FIG. 21A is a perspective view of FIG. 21;
FIG. 21B is a top view of FIG. 21;
FIG. 22 is a partial anterior/posterior view of the nail implant assembly of FIG. 1 showing the screw in a greater trochanter to lesser trochanter position;
FIG. 22A is a partial anterior/posterior view of another embodiment of the present invention with a fully threaded cortical screw to connect the greater trochanter to the lesser trochanter;
FIG. 23 is a partial anterior/posterior view of the nail implant assembly of FIG. 1 showing the screw bridging a transverse neck fracture;
FIG. 24 is a partial anterior/posterior view of the nail implant assembly of FIG. 1 showing the screw bridging a partially longitudinal neck fracture;
FIG. 25 is a partial anterior/posterior view of the nail implant assembly of FIG. 1 showing the nail in a retrograde position with the screw in a transverse direct position in the distal femur;
FIG. 26 is a plan view of a radiograph showing a femur in an anterior/posterior view;
FIG. 27 is a perspective view of a nail kit including a fixture for positioning the bushing of a nail according to yet another embodiment of the present invention;
FIG. 27A is a partial perspective view of the nail kit of FIG. 27;
FIG. 28 is a partial top view of the nail kit of FIG. 27;
FIG. 28A is a partial top view of the nail kit of FIG. 27;
FIG. 29 is an anterior/posterior view of a trochanteric intramedullary nail assembly according to another embodiment of the present invention;
FIG. 30 is a medial/lateral view of the intramedullary nail assembly of FIG. 29 showing the bow in the middle portion of the nail;
FIG. 31 is an anterior/posterior view of a retrograde nail implant assembly according to another embodiment of the present invention;
FIG. 32 is a partial anterior/posterior view of the intramedullary nail assembly of the retrograde nail implant assembly of FIG. 31 showing the locking plug for securing the bushing of the nail;
FIG. 33 is an anterior/posterior view of a fusion nail implant assembly according to another embodiment of the present invention;
FIG. 34 is a partial anterior/posterior view of the intramedullary nail assembly of the fusion nail implant assembly of FIG. 33 showing the locking plug for securing the bushing of the nail;
FIG. 35 is a partial anterior/posterior view of a tibial nail implant assembly according to another embodiment of the present invention;
FIG. 36 is a partial anterior/posterior view of a humeral nail implant assembly according to another embodiment of the present invention;
FIG. 37 is a partial medial/lateral view of a trochanteric intramedullary nail assembly with a plug being constructed by a transverse end cap according to another embodiment of the present invention;
FIG. 38 is a anterior/posterior view of the nail assembly of FIG. 37;
FIG. 39 is a plan view of the transverse end cap of the intramedullary nail assembly of FIG. 37;
FIG. 40 is a plan view of a fastener for use with the end cap of FIG. 39;
FIG. 41 is a partial medial/lateral view of a nail implant assembly according to yet another embodiment of the present invention showing a intramedullary nail with a translating bushing;
FIG. 42 is a partial top view of FIG. 41 showing the translating bushing and the nail;
FIG. 43 is a perspective view of a proximal locking plug for use with the intramedullary nail of FIG. 41;
FIG. 44 is a plan view of a distal locking plug for use with the intramedullary nail of FIG. 41;
FIG. 45 is a partial medial/lateral view of intramedullary nail assembly according to yet another embodiment of the present invention in the form of an intramedullary nail assembly with a two spaced-apart bushing;
FIG. 46 is a partial anterior/posterior view of a nail implant assembly according to yet another embodiment of the present invention utilizing the intramedullary nail assembly of FIG 45;
FIG. 47 is a partial anterior/posterior view of the nail implant assembly of FIG. 46 for use to repair both neck fractures and greater trochanter to lesser trochanter fractures;
FIG. 48 is a partial anterior / posterior view of a nail implant assembly according to yet another embodiment of the present invention utilizing a locking plug and bushing set with pre-established preset position in a first plane;

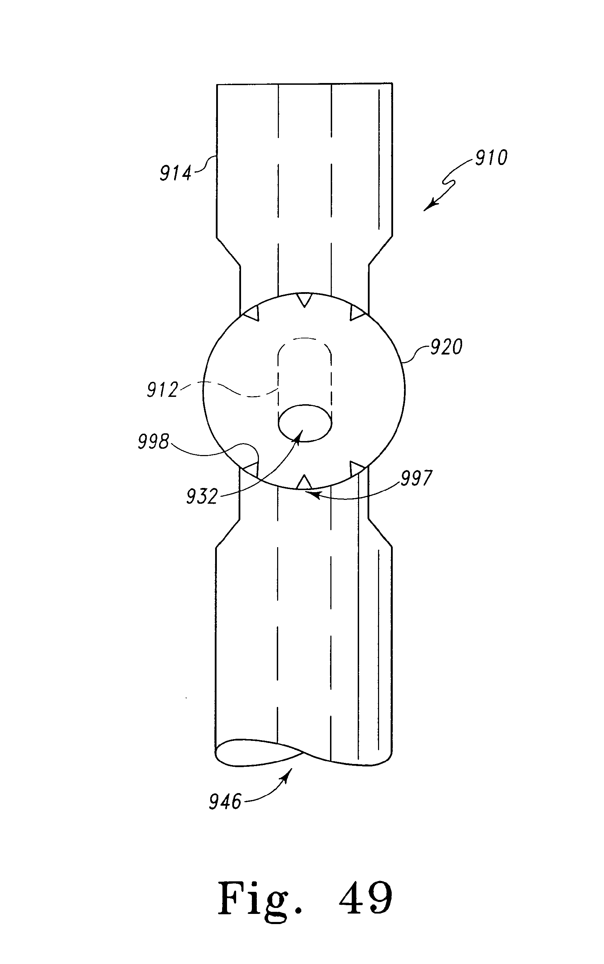

FIG. 49 is a partial medial/lateral view of the nail implant assembly of FIG. 48 showing present positions in a second plane;
FIG. 50 is a partial medial/lateral view of an intramedullary nail assembly according to yet another embodiment of the present invention utilizing a bushing with a transverse support cradles;
FIG. 51 is a partial anterior/posterior view of the intramedullary nail assembly of FIG. 48;
FIG. 52 is a plan view of the transverse support cradle of the nail assembly of FIG. 50;
FIG. 53 is a partial medial/lateral view of an intramedullary nail assembly according to yet another embodiment of the present invention utilizing a snap-in bushing;
FIG. 54 is a partial anterior/posterior view of the nail implant assembly of FIG. 50;
FIG. 55 is a flow diagram of a method of performing trauma surgery in accordance with yet another embodiment of the present invention;
FIG. 56 is a flow diagram of a method of performing trauma surgery in accordance with yet another embodiment of the present invention; and
FIG. 57 is a flow diagram of another method of performing trauma surgery in accordance with yet another embodiment of the present invention.

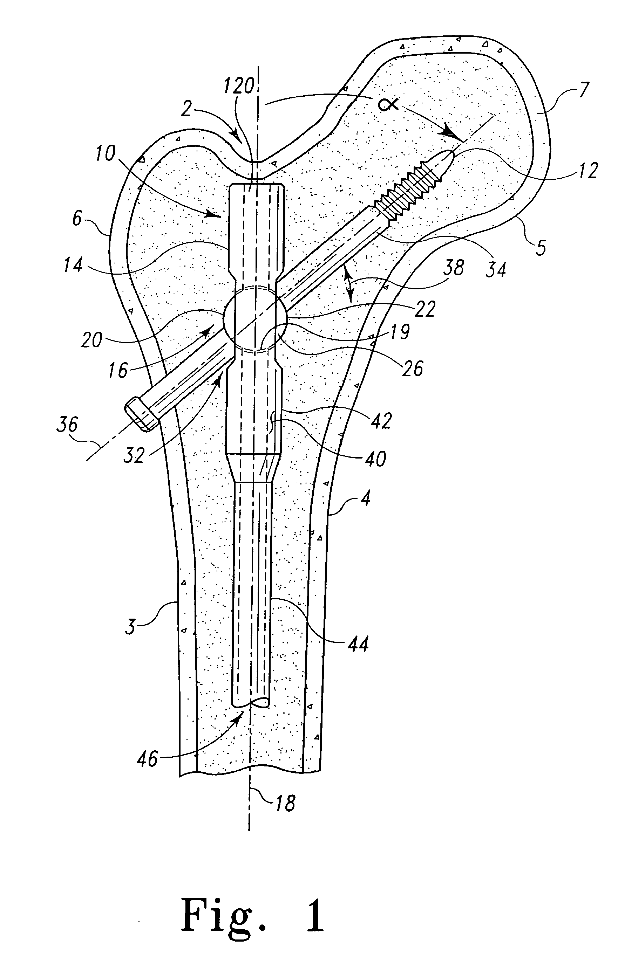

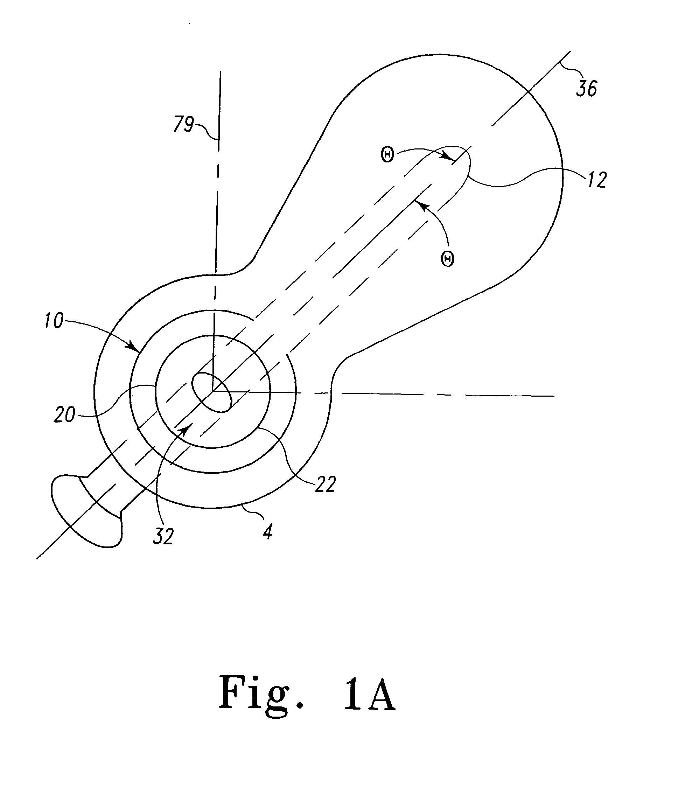

Referring to the drawings, FIG. 1 shows an intramedullary nail assembly 10 for use in the intramedullary canal 2 of a long bone 4. The long bone 4 may be any long bone of the body, for example, a femur, tibia, or a humerus.

The intramedullary nail assembly 10 is used with a screw 12 in an intramedullary canal 2 of a long bone 4. The nail assembly 10 includes a nail 14. The nail 14 is adapted for positioning at least partially in the medullary canal 2. The nail 14 defines an aperture 16 through the nail 14. The nail 14 defines a longitudinal axis 18 of the nail 14.

The nail assembly 10 further includes a bushing 20. The bushing 20 is positioned at least partially in the aperture 16 of the nail 14. The bushing 20 is adapted to receive the screw 12 in a plurality of angular positions with respect to the longitudinal axis 18 of the nail 14.

The bushing 20 may be adapted to receive the screw 12 in a plurality of angular orientations in a variety of manners or embodiments. For example, the nail assembly 10 may be adapted such that the orientation of the bushing 20 within the aperture 16 of the nail 14 can be adjusted.

The bushing 20 may be rotatably positioned within the nail 14 in any of various suitable configurations. For example, the bushing 20 may rotate about periphery 22 of the bushing 20. The bushing 20 may have any shape and may, for example, be spherical. For example, rotational centerline 26 of bushing 20 may remain in a first position. The fixed position of the centerline 26 of the bushing 20 may be accomplished by concave cradle 19 formed in the nail 14. The bushing 20 further includes a transverse bushing opening 32 for receiving shank 34 of the screw 12. The transverse bushing opening 32 may have any orientation if the bushing 20 is spherical.

The transverse opening 32 defines a transverse opening centerline 36, which forms an angle α with the longitudinal opening 18 of the nail 14. The angle α may be altered or adjusted to obtain the optimum angle α by rotating the bushing 20 in the direction of arrows 38. The clearance between the bushing 20 and the nail 14 may be minimal to maintain the angle α once established.

Referring now to FIG. 1A, the nail assembly 10 is shown in a top view installed in the femur 4. The nail assembly 10 includes the nail 14 to which the bushing 20 is rotatably secured. The screw 12 slidably fits through transverse bushing opening 32 of the bushing 20. The screw 12 defines a screw centerline 36, which because of the spherical shape of the bushing 20, may be able to rotate in the direction of arrows 24 at an angle θ in each direction from the centerline 36.

The nail 14 may have any suitable shape such that the nail 14 may be fitted into the canal 2 of the long bone 4. For example, the nail 14 may have an outer periphery 40 that may, for example, be cylindrical or round. The periphery 40 of the nail 14 may be uniform or may have, as is shown in FIG. 1, a larger diameter near condylar portion 6 of the femur 4. The periphery 40 may have a larger diameter at proximal portion 42 of the nail and a smaller diameter at distal portion 44 of the nail 14. The nail 14 may further have a solid cross section or may, as is shown in FIG. 1, be cannulated or include a longitudinal opening 46 extending along centerline 18 of the nail 14. The nail 14 may be straight or linear or may be bent or curved to conform to the medullary canal 2 of the long bone 4.

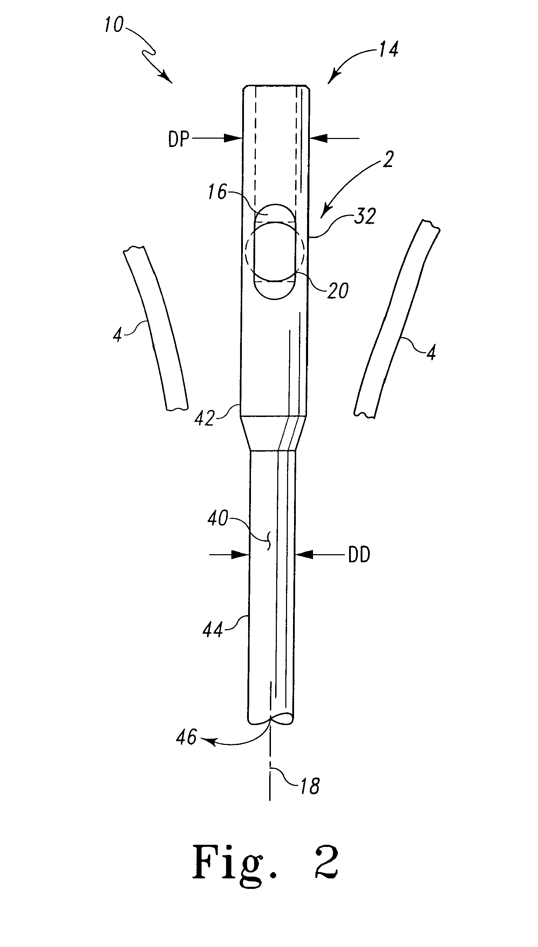

For example, and as shown in FIG. 2, the shape of the periphery 40 of the nail 14 is shown in greater detail. As shown in FIG. 2, the nail 14 has a generally circular cross section. The nail 14 includes the proximal portion 42, which is defined by diameter DP, as well as a distal portion 44 extending from the proximal portion 42 and having a circular cross section with a diameter DD.

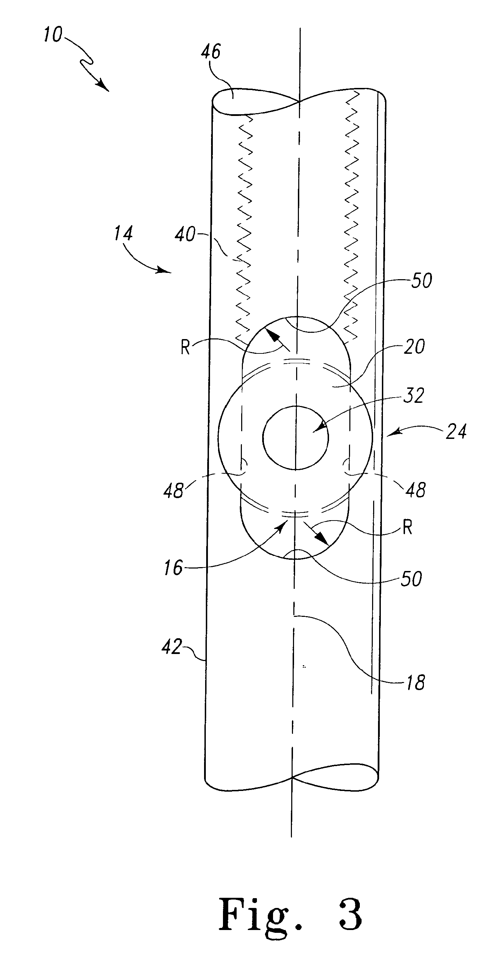

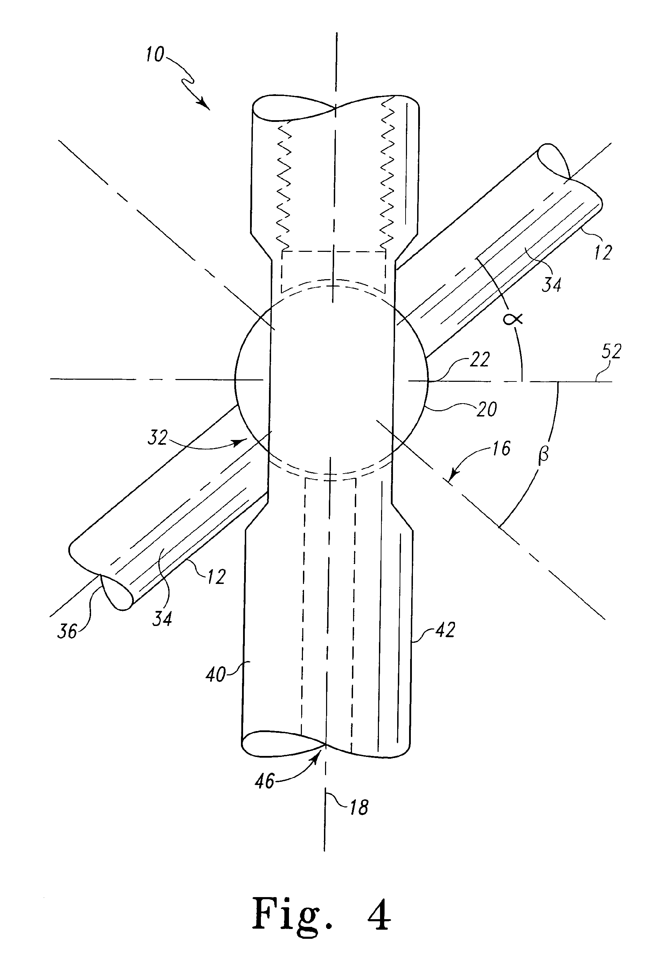

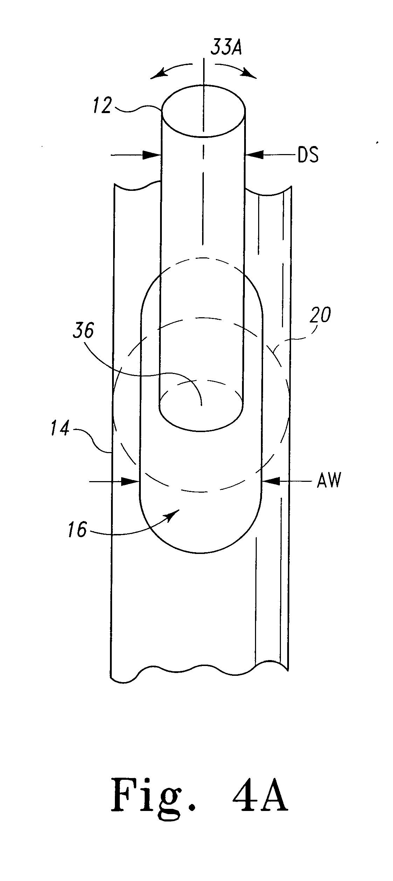

Referring now to FIGS. 3 and 4, the bushing 20, which is positioned in the nail 14 to form the nail assembly 10 is shown in greater detail. As shown in FIGS. 3 and 4, the bushing 20 is fitted into aperture 16 formed in the nail 14. The aperture 16 is preferably large enough to receive the bushing 20 to permit the screw 12 (see FIG. 1) to be placed in a plurality of angular positions with respect to longitudinal axis 18 of the nail 14.

For example, and as shown in FIG. 3, the aperture 16 has a generally constant cross section and is defined by opposed planar ends 48. The aperture 16 as shown in FIG. 3 is further defined by opposed semi-circular ends 50 extending from the planer ends 48.

Referring now to FIG. 4, the shape of the aperture 16 permits the screw 12 to rotate about centerline 26 of bushing 20 in an arcuate direction and an angle of, for example, α from the horizontal centerline 52 to centerline 36 in the proximal direction and in an angle β from the horizontal centerline 52 to the centerline 36 of the screw 12 in the distal direction.

Referring now to FIG. 4A, the nail assembly 10 is shown in the medial/lateral plane. The nail assembly 10 includes the nail 14 to which the spherical bushing 20 is pivotally connected. The bushing 20 includes a through opening 32 through which screw 12 is slidably fit. The bushing 20 is fitted into aperture 16 formed in the nail 14 as shown in FIG. 4A, so that the screw 12 may move in the direction 33A from the centerline 36 of the screw 12. The aperture 16 has a width AW, which is wider than diameter DS of the shank of the screw 12, so that the screw 12 may rotate in the direction of arrows 33A to provide for motion in the medial/lateral direction as shown in FIG. 4A.

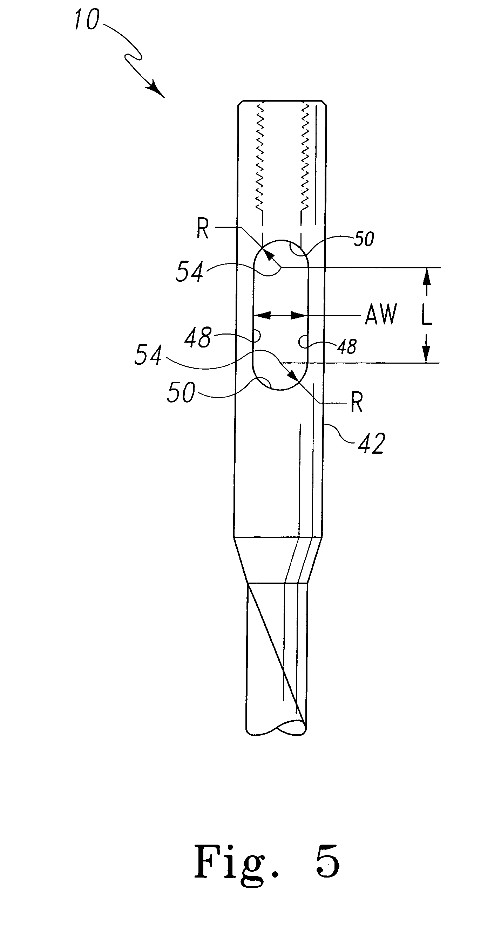

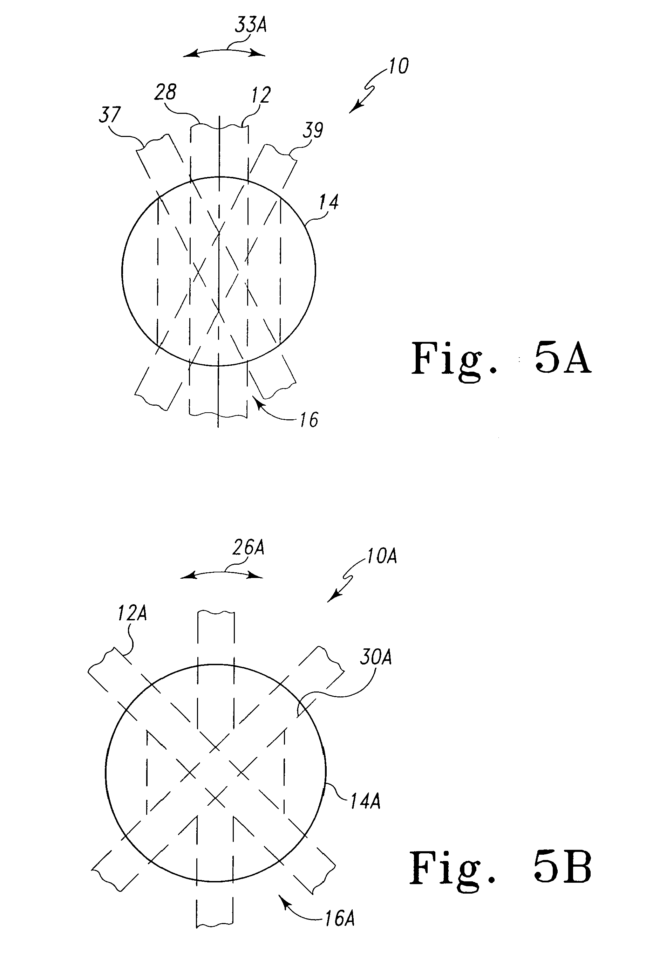

Referring now to FIG. 5, the aperture 16 is shown in greater detail. The aperture 16 may be defined, for example, by a width W extending from one of the opposed planer ends 48 to the other of the opposed planer ends 48. The aperture 16 may further be defined by a pair of opposed radii R defining the opposed semi-circular ends 50. The radii R extend from origins 54. The origins 54 are separated by a distance L.

Referring now to FIG. 5A, the nail assembly 10 is shown in a top view. The nail assembly 10 defines the aperture 16 through which the screw 12, as shown in solid in first position 28, is shown. The screw 12 may be rotated in the direction of arrow 33A from first position 28 to, for example, second position 37 and also to third position 39. As described earlier, the screw 12 may be rotated in the direction of arrows 33A by an angle θ in both directions from the first position 28, because the width of the aperture 16 is wider than the diameter DS of the screw 12.

Referring now to FIG. 5B, yet another embodiment of the present invention is shown as nail assembly 10A. Nail assembly 10A is similar to nail assembly 10 of FIGS. 1-5, except that nail assembly 10A includes a nail 14A having chamfers or flats 30A positioned adjacent the aperture 16A. The flats 30A serve to permit additional motion in the direction of arrows 26A, so that the screw 12A may rotate in a larger angle or arc with respect to the centerline 18A of the nail 14A.

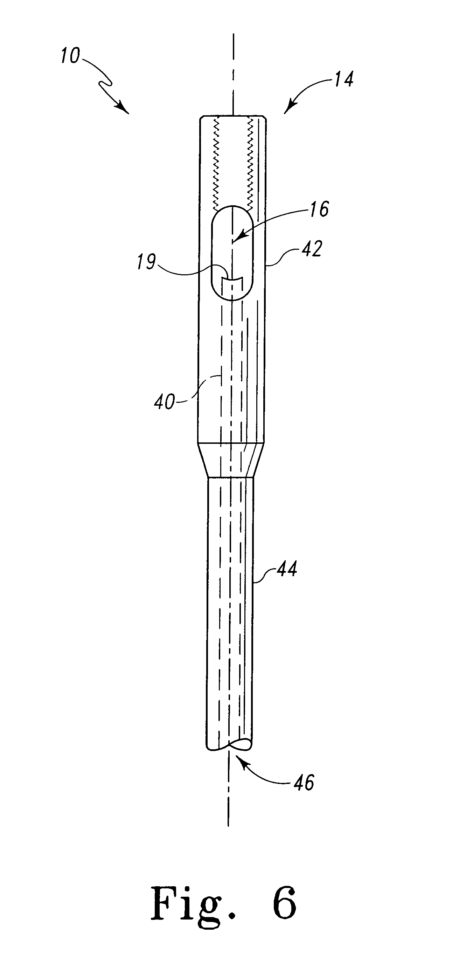

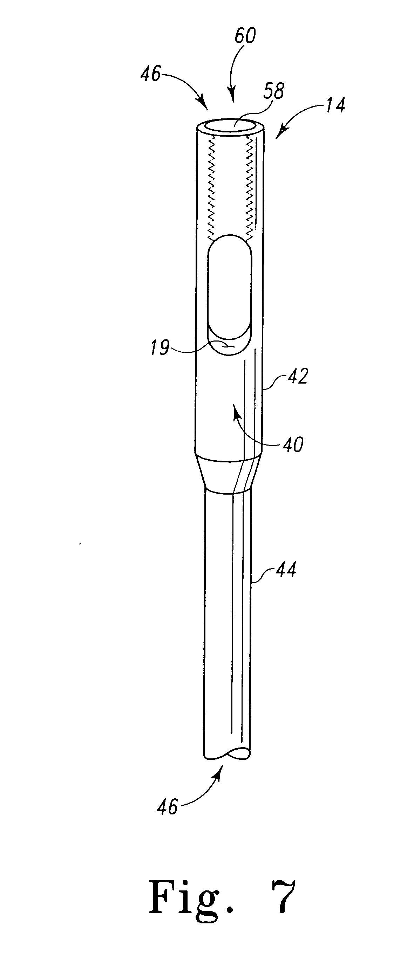

Referring now to FIGS. 6 and 7, the proximal portion 42 of the nail 14 of nail assembly 10 is shown with the bushing 20 not installed into the nail. The nail 14 includes the aperture 16 for receiving the bushing 20 as well as cradle 19 for supporting periphery 22 of bushing 20. The nail 14 may be solid or may be, as shown in FIGS. 6 and 7, cannulated. The nail 14 includes the longitudinal opening 46 extending along longitudinal centerline 18 of the nail 14.

The multiple position nail assembly of the present invention preferably includes means for providing selectively rigidly connection of the angular position of the bushing with respect to the nail, such that the nail assembly may support the neck of, for example, the femur.

The bushing 20 may be selectively rigidly connected to the nail 140in any suitable manner. For simplicity, the periphery 22 of the bushing 20 may be selectively rigidly or rotatably in contact or in engagement with the cradle 19 of bushing 20 to rigidly connect the bushing 20 to the nail 14

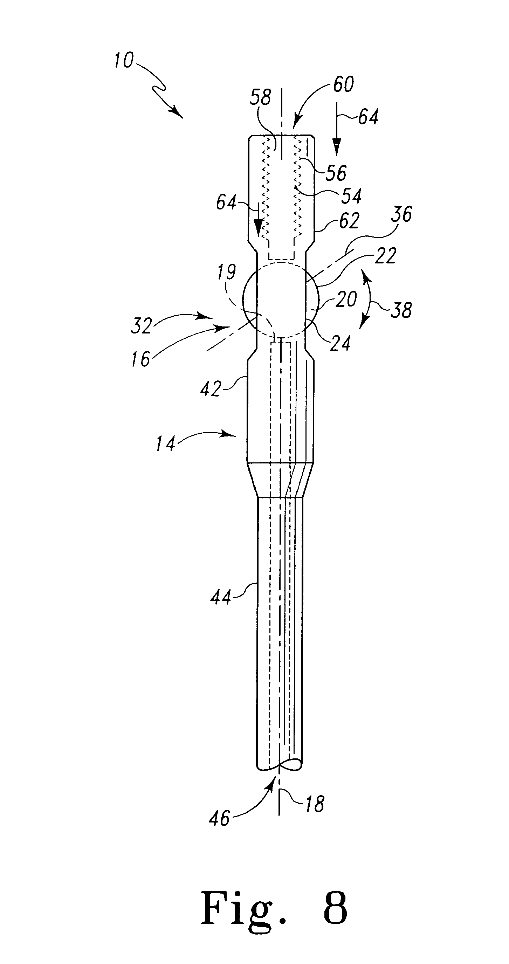

For example, and as shown in FIG. 8, locking means 54 in the form of, for example, a locking plug is used to selectively rigidly position the bushing 20 with respect to the nail 14. The locking plug 54 is selectively urged into contact with, for example, periphery 22 of the bushing 20. The locking plug 54 may engage the bushing 20 selectively by any means. For example, and as shown in FIG. 8, locking plug 54 may include external threads 56, which engage with internal threads 58 formed on counter-bore 60 formed in the proximal end of proximal portion 42 of the nail 14. The locking plug 54 includes a stem 62, which is in selectable contact with periphery 22 of the bushing 20.

For example and as shown in FIG. 8, the centerline 36 of the bushing opening 32 is rotated in the direction of arrows 38 such that the centerline 36 is in an appropriate angular position. Once the bushing opening 32 is properly oriented, the locking plug 54 is rotated such that the stem 62 is advanced in the direction of arrow 64 such that the stem 62 locks against the periphery 22 of the bushing 20 providing for a fixed angular orientation of the bushing opening 32 with respect to the nail 14.

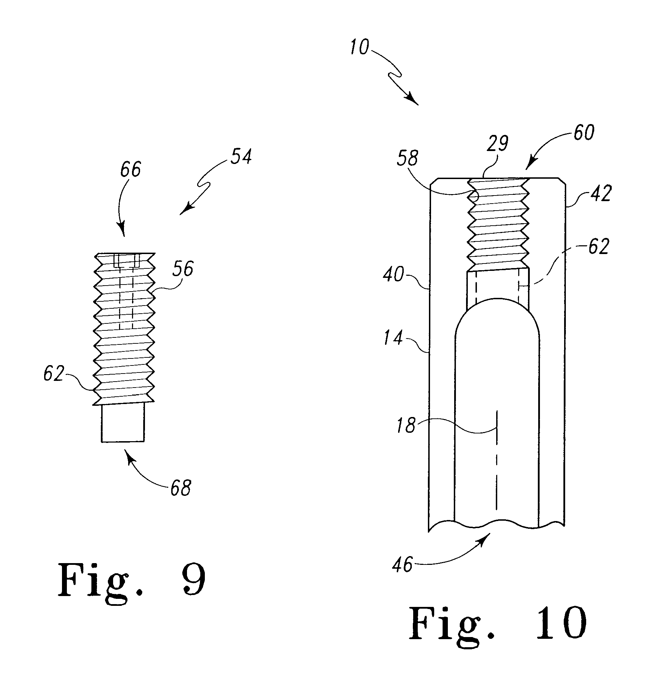

Referring now to FIG. 9, the locking plug 54 is shown in greater detail. The locking plug 54 includes external threads 56 for cooperating with the internal threads 58 of the nail 14 (see FIG. 7). The locking plug 54 further includes the stem 62 for contact with the bushing 20 (see FIG. 8). The locking plug 54 may further include a hexagonal drive 66 for cooperating with, for example, a standard screw driver (not shown). The locking plug 54 may include a central longitudinal opening 68.

Referring now to FIG. 10, the proximal portion 42 of the nail 14 of the nail assembly 10 is shown in greater detail. The proximal portion 42 may define longitudinal opening 46 extending along longitudinal centerline 18 of the nail 14. The nail 14 may include a generally cylindrical nail periphery 40 as well as a counter bore 60 extending from first end 29 of the nail 14. The counter bore 60 may include internal threads 58 formed on the outer diameter of the counter bore 60. The internal threads 58 are adapted for receiving the plug 54.

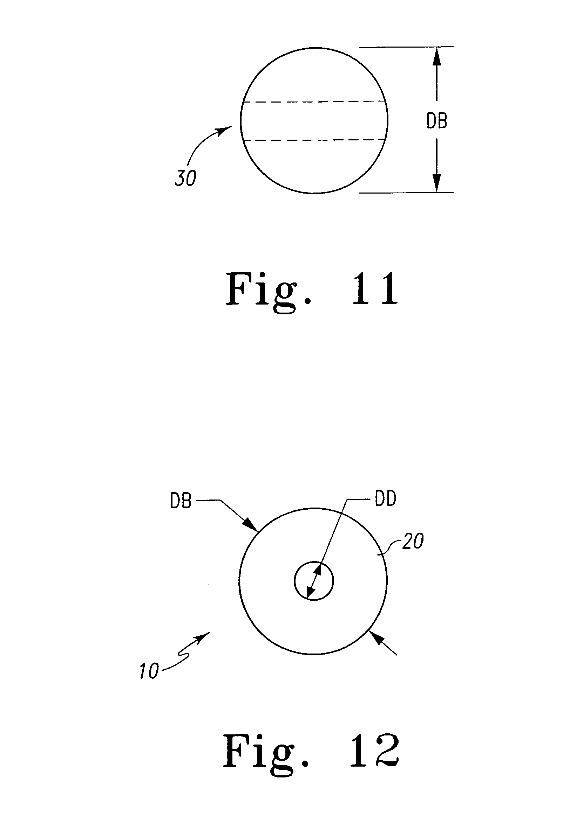

Referring now to FIG. 11, the bushing 20 is shown in greater detail. The bushing 20 is defined by a diameter DB. The bushing 20 may include central opening 32.

Referring now to FIG. 12, the bushing 20 may have a generally spherical shape and a circular cross section and be defined by diameter DB. The central opening 32 in the bushing 20 may be defined by a diameter DO.

The nail assembly 10 of FIGS. 1-3 may be made of any suitable durable material compatible with the human body. For example, the nail assembly 10 may include components, for example, the nail 14, the bushing 20, as well as the locking plug 54 may be made of, for example, a metal, a plastic or a composite material. If made of a metal, the components of the nail assembly 10 may be made of, for example, a cobalt chromium alloy, a stainless steel alloy or a titanium alloy. The components of the nail assembly 10 may, for simplicity and to avoid material interactions, be all made of the same material.

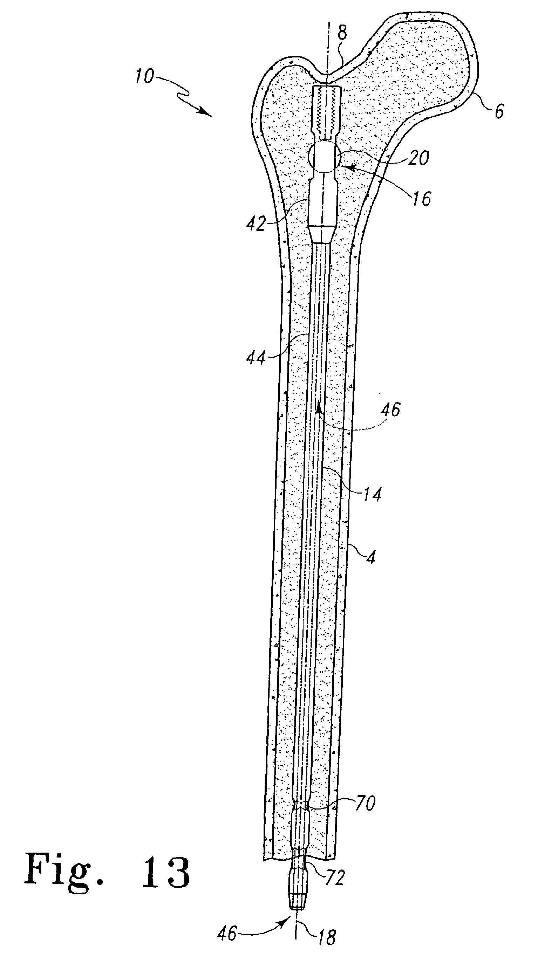

Referring now to FIG. 13, the entire nail assembly 10 is shown. While it should be appreciated that the nail and nail assembly of the present invention may be utilized in any long bone and for any of the various commercially types of intramedullary nails in the long bones of the body.

The nail assembly 10 as shown in FIG. 13, is in the form of a piri forma nail. The piri forma nail 14 of the nail assembly 10 of FIG. 13 is designed to be inserted through the piri forma 8 of the condylar portion 6 of the femur 4. The piri forma 8 of the femur 4, as is shown in FIG. 13, is in line with the longitudinal centre of the femur 4 as viewed in the anterior/posterior view of FIG. 13. Thus, the nail 14 of the nail assembly 10 has a longitudinal centerline 18, which as shown in the anterior/posterior view is generally straight.

The nail assembly 10 includes the proximal portion 42 and the distal portion 44. The proximal portion 42 includes the bushing 20, which is secured in cradle 19 to nail 14. The nail 14 may include the longitudinally extending opening or cannula 46.

The nail 14 further includes additional transverse openings for securement of screws in the distal portion 44 of the femur 4 to properly secure the nail assembly 10 to the femur 4. The nail 14 may include a singular, or as is shown in FIG. 13, a plurality of distal openings. For example, the nail 14 includes a first distal opening 70, which as is shown in FIG. 13, may extend from the medial to lateral direction, as well as a second distal opening 72, which may also extend from a medial to lateral direction.

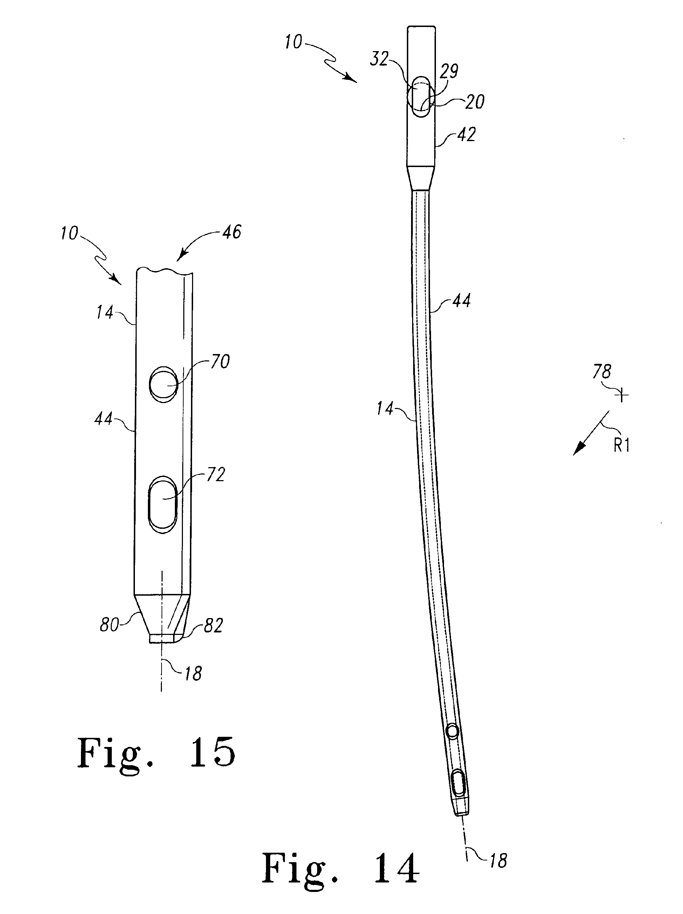

As shown in FIG. 14, and to accommodate the natural curve or arcuate nature of the femur, the nail 14 of the nail assembly 10 has a generally arcuate shape when viewed in the medial/lateral view of FIG. 14. The longitudinal centerline 18 of the nail 14, as is shown in FIG. 14, extends in a direction defined by radius R1 extending from origin 78. The radius R1 and the position of origin 78 are selected to model the shape of the human femur. It should be appreciated that depending on the size of the nail 14 and the particular anatomical sutures of the femur that the nail 14 is designed to accommodate, the position of origin 78 of radius R1 and the dimension of R1 will be correspondingly changed.

Referring now to FIG. 15, the distal openings 70 and 72 in the distal portion 44 of the nail 14 are shown in greater detail. It should be appreciated that the first opening 70 and the second opening 72 may have any suitable shape. The openings 70 and 72 may be perpendicular or transverse to the longitudinal axis 18 of the distal portion 44 of the nail 14. Such transverse orientation of the openings 70 and 72 provides an ability for transverse screws (not shown) to be secured through the openings and into the cortical walls of the femur.

The openings 70 and 72 may have any suitable shape and may, as shown in FIG. 15, include cylindrical openings such as shown in the first opening 70 or provide for oval openings such as shown in second opening 72. The oval openings, such as the second opening 72, may permit the distal portion 44 of the nail 14 to move axially relative to the bone or screw. To assist in the installation of the nail assembly 10 into the medullary canal 2, the nail 14 may include a tapered flat 80 extending from end 82 of the nail 14. The flat 80 contacts the inner wall of the canal 2 during installation.

While the nail assembly 10 of the present invention may include, as shown in FIGS. 14 and 15, two distal openings, it should be appreciated that the nail assembly may include additional openings to accommodate additional distal screws or to accommodate distal screws at a variety of angular orientations with respect to the longitudinal axis 18 of the nail 14.

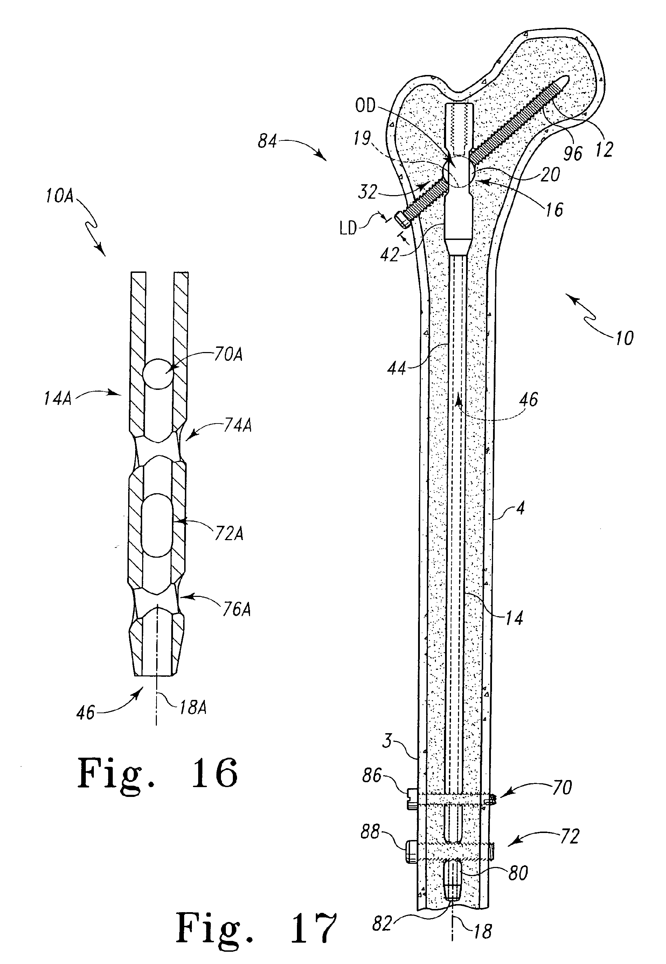

For example, and as shown in FIG. 16 and according to another embodiment of the present invention, nail assembly 10A is shown. The nail assembly 10A is similar to nail assembly 10 of FIGS. 1-3, and includes a nail 14A, which has four distal openings. For example, and as shown in FIG. 16, the nail assembly 10A includes a first opening 70A similar to the first opening 70 of the nail 14 of FIG. 15, as well as a second opening 72A in the form of a slot similar to the opening 72 of the nail 14 of FIG. 15.

The nail 14A may, as is shown in FIG. 16, further include a third opening 74A and a fourth opening 76A. The third opening 74A and the fourth opening 76A may, as is shown in FIG. 16, be, for example, transverse or perpendicular to the longitudinal axis 18A of the nail 14A and perpendicular to the first opening 70A and the second opening 72A. The third opening 74A and the fourth opening 76A may, as is shown in FIG. 16, be generally cylindrical and may, and as shown in FIG. 16, be slightly skewed from a pure perpendicular direction with respect to the longitudinal axis 18A.

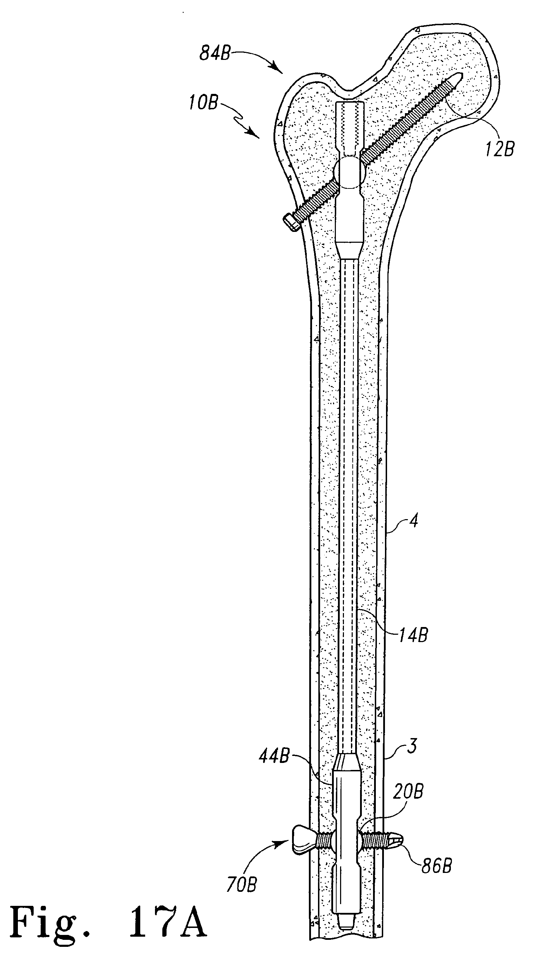

Referring now to FIG. 17, a nail implant assembly 84 according to another embodiment of the present invention is shown. The nail implant assembly 84 includes nail assembly 10 as well as screw 12. The nail implant assembly 84, as shown in FIG. 17, may also include distal screws. For example, the distal screws may include a first distal screw 86 for cooperation in first distal opening 70 as well as a second distal screw 88 for cooperation with the second distal opening 72. The first distal screw 86 and the second distal screw 88 may, for example, be in the form of cortical screws for engaging cortical bone 3 of the femur or long bone 4.

Referring now to FIG. 17A, a nail implant assembly 84B according to another embodiment of the present invention is shown. The nail implant assembly 84B includes nail assembly 10B as well as screw 12B. The nail implant assembly 84B, as shown in FIG. 17A, may also include a distal screw bushing 20B for receiving distal screw 86B through distal screw opening 70B formed in distal screw bushing 20B secured to distal portion 44B of nail 14B. For example, the distal screw 86B may, for example, be in the form of a cortical screw for engaging cortical bone 3 of the femur or long bone 4.

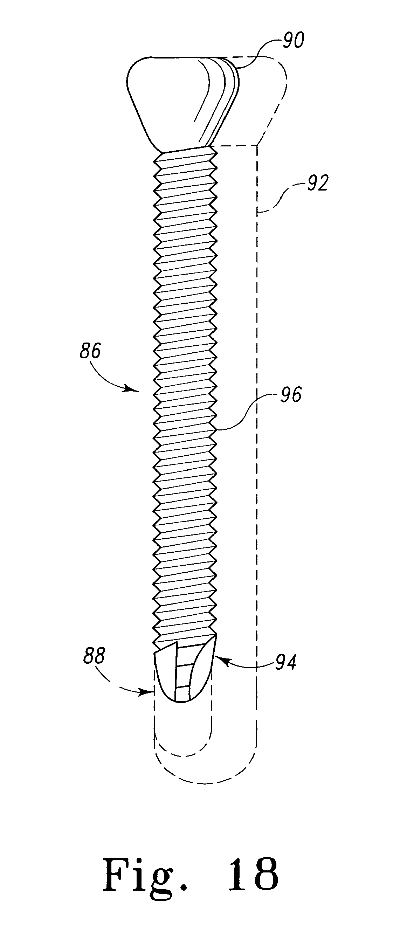

Referring now to FIG. 18, the distal screws 86 and 88 are shown in greater detail. The distal screw 86 includes a head 90 from which cortical fine pitch threads 92 extend. The screw 86 further includes a self-drilling and self-tapping portion 94 opposed to the head 90. The second distal screw 88 is similar to the first distal screw 86 and varies in its length as shown in phantom.

Referring to FIG. 18 and as shown in dashed lines, the nail assembly 10 may further include a large proximal cortical screw 92. The screw 92 may be used for greater trochanter to lesser trochanter attachment.

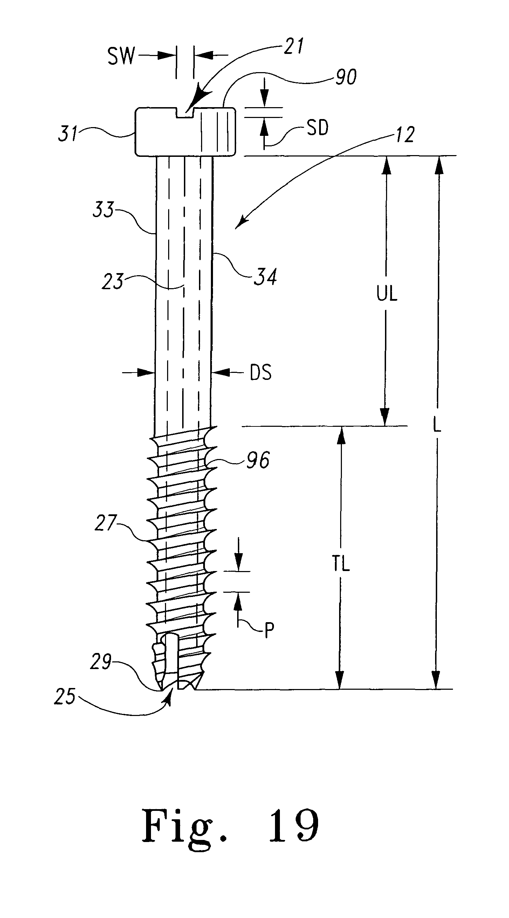

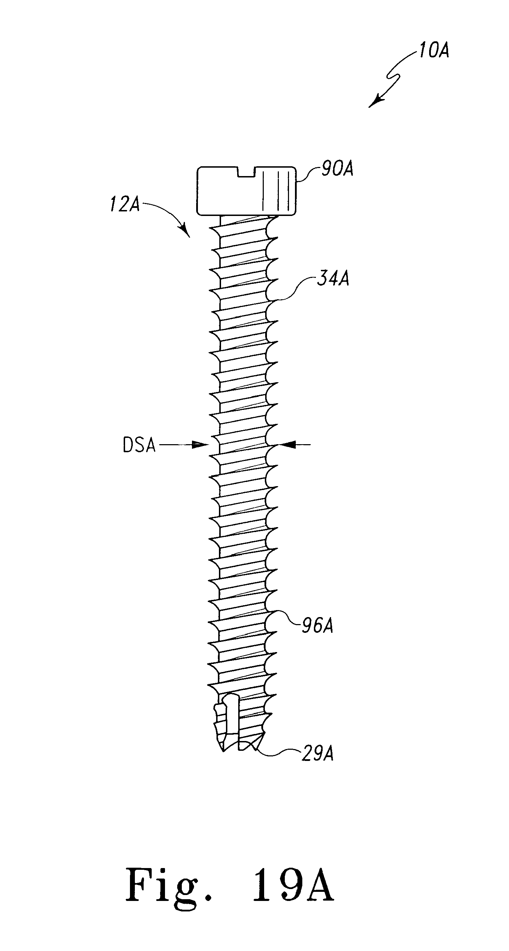

Referring now to FIG. 19, the screw 12 for use with nail assembly 10 of FIGS. 1-3 is shown. The screw 12 includes the shank portion of which defines threads 96. The screw 12 may be any screw which can be fitted in the aperture 32 of the bushing 20 of the nail 14 and capable of being adapted to be securely fitted to the nail 14. For example and as is shown in FIG. 19, the screw 12 may include the head or lip 90 extending from the shank 34.

The lip 90 may be designed to prevent the screw 12 from migrating through the opening 32 of the bushing 20. The lip 90 may have any suitable size and shape capable of preventing the screw 12 from transversing out of the opening 32. For example, the lip 90 may have a lip diameter LD, which is larger than the opening diameter OD of the opening 32.

Referring now to FIG. 19, the screw 12 may include or define a rotating feature in the form of, for example, slot 21 formed in the screw 12. The slot 21 may have any suitable size. The slot 21 may be utilized to assist in rotating the screw 12 and as such may be centrally located about longitudinal centerline 23 of the screw 12. The slot 21 may have a slot width SW as well as a slot depth of SD. The slot 21 may include a radius located in the slot 21 to reduce stress risers caused by the slot 21. The slot width SD and slot length SL are designed to be sufficient for the screw 12 to cooperate with, for example, a screwdriver (not shown) for implanting the screw 12 into the long bone 4. An internal or external hexagonal or rectangular feature (not shown) may be substituted for the slot 21.

As shown in FIG. 19, the screw 12 may be cannulated and include a longitudinal opening 25 extending along longitudinal centerline 23 of the screw 12. The longitudinal opening 25 may be utilized, for example, for receiving a guide wire (not shown) to guide the screw 12 into position within the opening 32 of the nail assembly 10 and to properly position the screw 12 into the long bone 4.

The screw 12 may further include a removal feature, not shown, in the form of, for example, internal threads formed in a small counter bore (not shown) formed in the longitudinal opening 25 adjacent the slot 21 of the screw 12. The screw 12 may further include a large counter bore (not shown) extending from the end of the screw 12 and may be concentric with the small counter bore as well as with the longitudinal opening 25.

As shown in FIG. 19, the screw 12 may further include a plurality of threads 96 formed on the shank 34 of the screw 12. The threads 96 may, as shown in FIG. 19, have a non-uniform cross-section, as disclosed in EP-A-1656899.

Referring again to FIG. 19, shank 34 of the screw 12 includes a first portion 27 into which the threads are formed. It should be appreciated that the first portion 27 may extend along the longitudinal axis 23 of the screw 12 from the first end 29 to second end 31 of the screw 12. It should also be appreciated and as is shown in FIG. 19, that the shank 34 may include a second portion 33. The second portion 33 of shank 34 may define a smooth surface. As is shown in FIG. 19, the shank 34 may be generally cylindrical and defined by a diameter, for example, DS.

The screw 12 as is shown in FIG. 19, is generally cylindrical and defined by diameter DS and an overall length L. The shank 34 of the screw 12 includes the first portion 27 which include threads 96 and the second portion 33 having the smooth surface. The overall length L, of the diameter DS is divided into a thread TL and a smooth or unthreaded length UL. The thread length TL defines the first portion 27 and the smooth length UL defines the second portion 33. The thread length TL may, for example, be a portion of, for example, 20-40% of the overall length L of the shank 34. It should be appreciated that the smooth length UL is preferably a sufficient length such that the second portion 33 of the screw 12 may be positioned in the oblique opening 32 of the bushing 20 of the intramedullary nail 14 (see FIGS. 1-3) to permit sliding compression of the bone fracture of femur 4.

The threads 96 as is shown in FIG. 19, may advance spirally around the shank 34 of the screw 12. The threads 96 may be defined by a pitch P defining a spacing along longitudinal axis 23 between adjacent threads. The threads 96 may advance spirally around the longitudinal axis 23 in either a right or a left hand spiral configuration. The threads may, as is shown in FIG. 19, be of a single lead type but may alternatively be double lead configuration or a triple lead configuration.

Referring now to FIG. 19A, a fully threaded cortical screw 12A is shown. The fully threaded cortical screw 12A may be used in nail assembly 10A similar to the nail assembly 10 of FIGS. 1-5. The screw 12A includes a shank 34A that, as shown in FIG. 19A, is fully threaded, i.e., the screw 12A includes a shank 34A including threads 96A extending around the periphery of the shank 34A of the screw 12A from head 90A to first end 29A of the screw 12A.

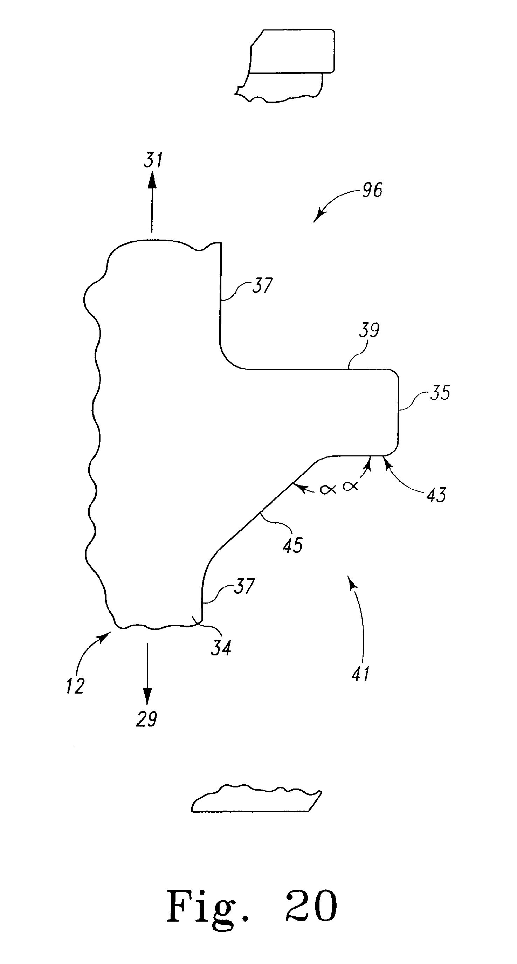

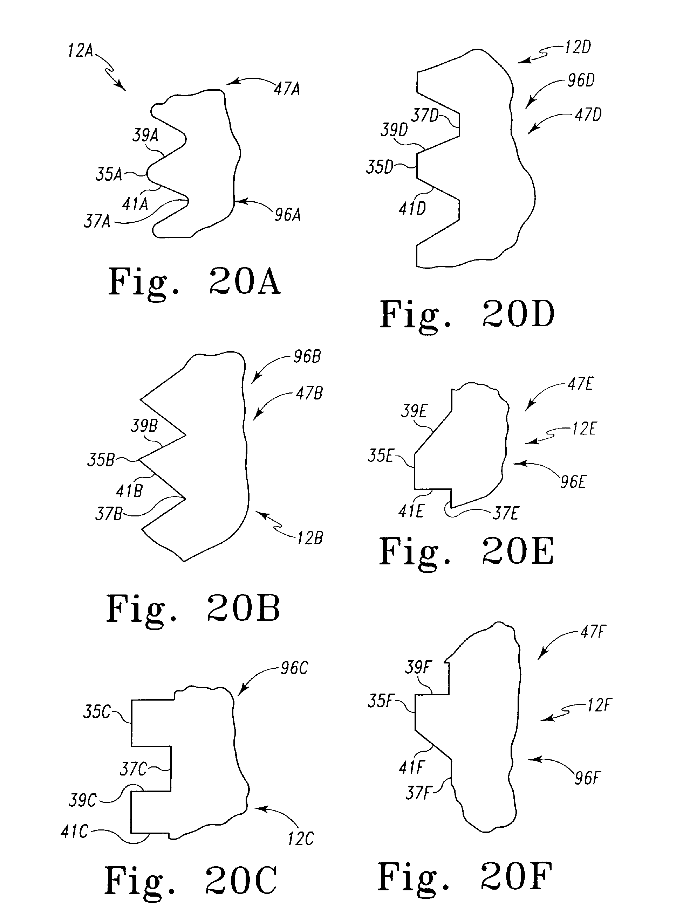

Referring now to FIG. 20, the threads 96 may have any suitable shape or thread form. For example and as shown in FIG. 20, the threads 96 may have a combination box and tapered configuration. For example and as is shown in FIG. 20, the threads 96 may have any suitable shape or profile. For example and is shown in FIG. 20, the profile may include a crest 35 and opposed root 37. A trailing surface 39 is positioned between the crest 35 and the root 37 adjacent second end 31 of the screw 12 while leading edge 41 is positioned between the crest 35 and root 37 adjacent the first end 29 of the screw 12.

As shown in FIG. 20, the leading edge 41 and the trailing edge 39 may be configured to provide for less force to assemble in the direction of arrow 43 than to disassemble in the direction opposed to arrow 43. Such ease of assembly and difficulty in disassembly may be accomplished as is shown in FIG. 20 by providing the trailing edge 39 with a configuration that is normal or perpendicular to the root 37 and the crest 35 while providing the leading edge 41 with a normal surface 43 and with an angled surface 45 between the normal surface 43 and the root 37.

The threads 96 of the screw 12 may, as is shown in FIG. 20, include the leading edge 41 such that the leading edge 41 includes normal surface 43 as well as an angled portion 45. The angled portion 45 provides for reduced force to assemble the screw 12 into the long bone or femur 4. The normal surface 43 and the angled portion 45 may define an angle αα between them. To minimize stress, the crest 35, the root 37, trailing surface 39, and leading edge 41 may include arcuate portions between them to minimize the stress.

Referring now to FIG. 20A-20F, alternative profile configuration for threads of the screw of the nail of the present invention is shown. Referring now to FIG. 20A, profile 47A is shown which includes arcuate roots and crest. For example and is shown in FIG. 20A, the profile 47A of the screw 12A includes an arcuate crest 35A to which the trailing angled surface 39A extends. The leading edge 41A extends likewise from the arcuate crest 35A. The profile 47A further includes an arcuate root 37A, which connects with trailing surface 39A and leading surface 41A.

Referring now to FIG. 20B, yet another profile for threads for screw of the present invention is shown as screw 12B includes threads 96B having a profile 47B which include generally v-shaped threads 96B. The profile 47B includes trailing surface 39B and leading surface 41B. The root 37B and the crest 35B are as shown in FIG. 20B are minimal.

Referring now to FIG. 20C, yet another profile of threads for a screw according to the present invention is shown. For example and is shown in FIG. 20C, the screw 12C includes threads 96C having a profile 47C that is blocked or rectangular. The profile 47C includes a trailing surface 39C and spaced-apart parallel leading surface 41C. The trailing surface 39C and the leading surface 41C are normal or perpendicular to root 37C and crest 35C.

Referring now to FIG. 20D, yet another embodiment of a profile of threads for a screw according to the present invention is shown. The profile 47D of threads 96D of the screw 12D has a generally truncated v-shape of that of a standard screw thread. The profile 47D includes a flat crest 35D and opposed angled trailing surfaces 39D and leading surface 41D. A root 37D extends from the trailing surface 39D and the leading surface 41D.

Referring now to FIG. 20E, yet another profile of threads of a screw of the present invention is shown as profile 47E. The screw 12E includes threads 96E having the profile 47E. The profile 47E includes a leading surface 41E that is normal to a crest 35E and a spaced apart parallel root 37E. The profile 47E further includes a trailing surface 39E that is positioned at an angle between the roots 37E and the crest 35E.

According to the present invention and referring now to FIG. 20F, yet another form of profile of the screw of the present invention. The screw 12F of FIG. 20F includes threads 96F defining profile 47F. The profile 47F includes a spaced apart parallel crest 35F and root 37F. The profile 47F includes a trailing surface 39F, which is normal to the root 37F and the crest 35F. The profile 47F further includes a surface 41F, which is positioned at angle between root 62F and crest 35F.

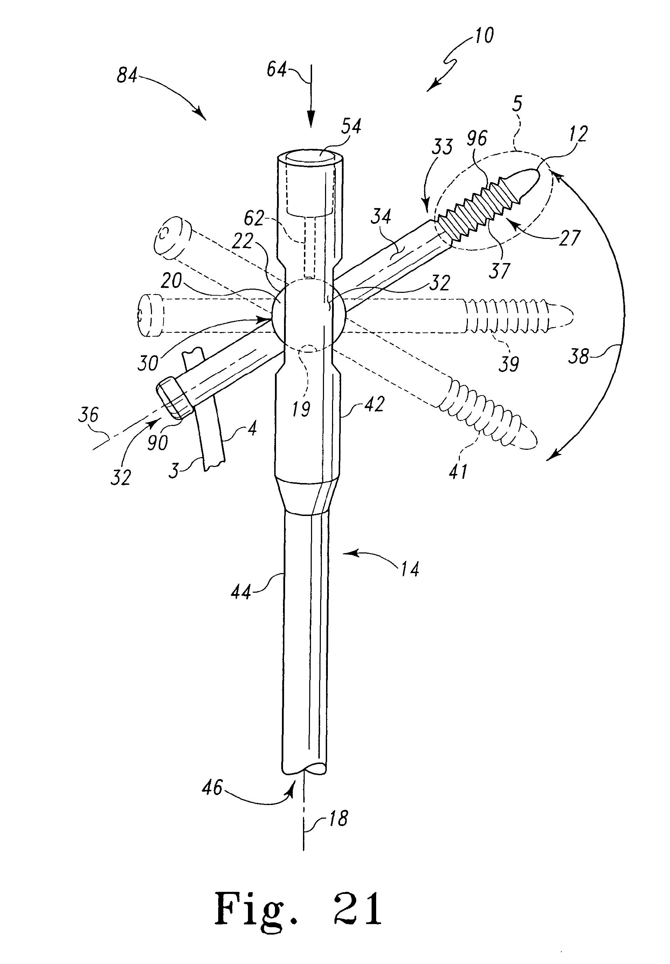

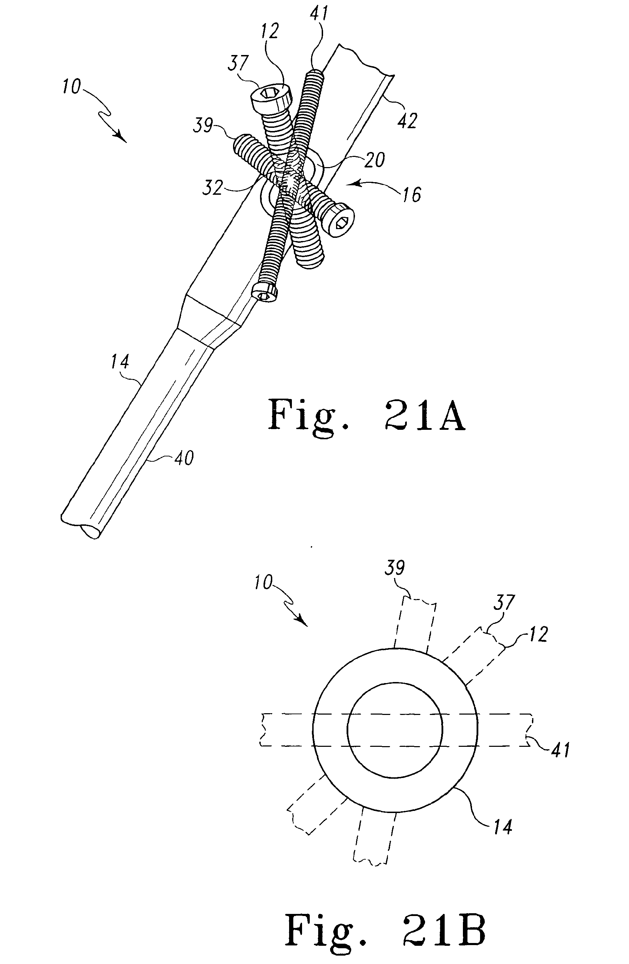

Referring now to FIG. 21, the proximal portion 42 of the nail 14 of the nail assembly 10 is shown in greater detail. The nail assembly 10 is shown with the screw 12 positioned in the nail assembly 10 to form the nail implant assembly 84. As shown in FIG. 21, the screw 12 includes shank 34, which is slidably fitted in bushing opening 32 formed in bushing 20.

The bushing 20 as shown in FIG. 21, is rotatably secured to the nail 14 by, for example, cradle 19 and locking pin 54, which cooperate with transverse opening 32 formed in the nail 14. As shown in FIG. 21, the screw 12 includes first portion 27, which includes the threads 96 and second portion 33, which is shown in FIG. 21, has a smooth periphery. The portion of the shank 34 of the screw 12, which is fitted in opening 32 of the bushing 20, is smooth to provide for sliding compression to assist in healing of the fracture, particularly the fracture of a femoral neck.

It should be appreciated however, that the threads 96 may extend over the entire shank 34 of the screw 12 and that the threads 96 may be fitted within the opening 32 of the bushing 20. Such a configuration may not be as conducive to sliding compression and may not provide the same degree of healing for a femoral neck fracture.

The capability of the bushing 20 to rotate in the direction of arrows 38 permits the longitudinal centerline 36 of the screw 12 to likewise rotate in the direction of arrows 38. Therefore, utilizing the nail assembly 10 of the present invention, the longitudinal centerline 36 of the screw 12 may be permitted to move from a first position 27 as shown in solid to a second position 33 as shown in the dashed line. The ability of the bushing 20 to rotate may further permit the centerline 36 of the screw 12 to move into third position 41 as shown in phantom.

It should be appreciated that the centerline 36 of the screw 12 may be positioned in any position between the first position 27 shown in solid and the third position 41 shown in phantom. By providing a nail assembly 10 that has a rotatable bushing 20, a wide variety of angular orientations of the screw with respect to the longitudinal centerline 18 of the nail 14 may be provided.

Once the bushing 20 is rotated into the proper position, such that the longitudinal centerline 36 of screw 12 is in the desired orientation, the locking plug 54 may be rotated such that the locking plate 54 advances in the direction of arrow 64, such that stem portion 62 of the locking plate 54 engages periphery 22 of the bushing 20. The stem portion 62 thereby locks the bushing 20 into a fixed angular orientation.

As shown in FIG. 21, when the screw 12 is in first position 37 as shown in solid, the head 90 of the screw 12 rests against the cortical bone 3 of the femur 4, and the second portion 33 of the shank 34 of the screw 12 passes through the opening 32 of the bushing 20. The threads 96 formed on the first portion 27 of the screw 12 engage with cancellous bone in neck 5 of the femur 4 and extend toward head 7 of the femur 4.

Referring now to FIG. 21A, the nail assembly 10 is shown in a perspective view. The nail assembly 10 includes the nail 14 having a smaller distal portion 44 and an enlarged proximal portion 42. The nail 14 defines the opening or aperture 16 into which the bushing 20 is pivotally secured. The bushing 20 includes a through opening 32 for slidably receiving the screw 12. As shown in FIG. 21A, the screw 12 may be presented in, for example, the first position 37 in solid. The screw 12 is also shown in second position 39 in dashed lines. The screw 12 is further shown in third position 41 in phantom. It should be appreciated that the orientation of the screw 12 may be varied in all three planes.

Referring now to FIG. 2 1 B, the screw 12 is viewed from the top of the nail 14. The nail assembly 10 includes the screw 12 that may be positioned in the first position 37 as shown in solid. The screw 12 may further be positioned in the second position 39 as shown in dashed lines. The screw 12 may further be positioned in, for example, third position 41 as shown in phantom.

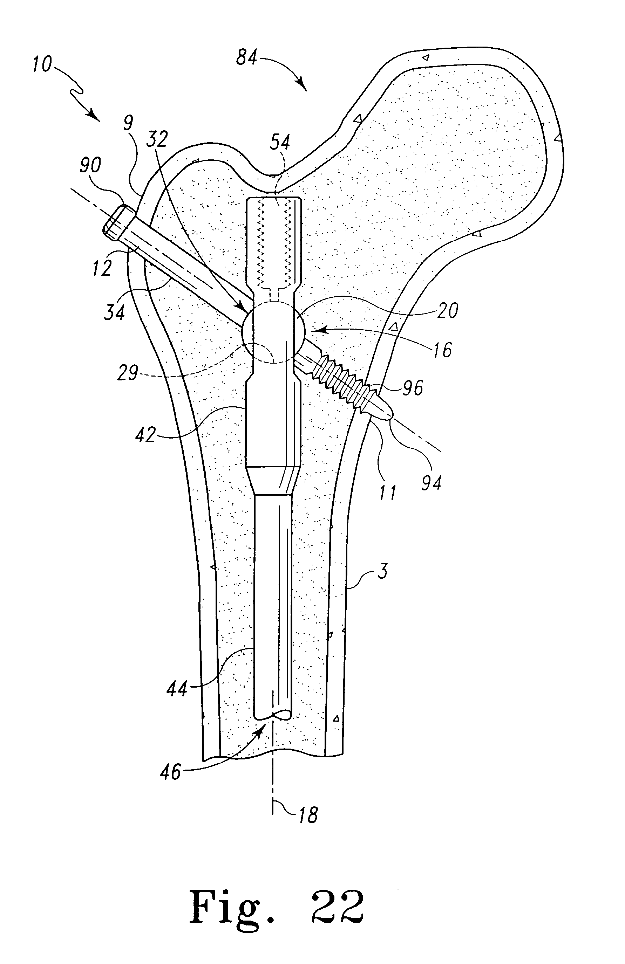

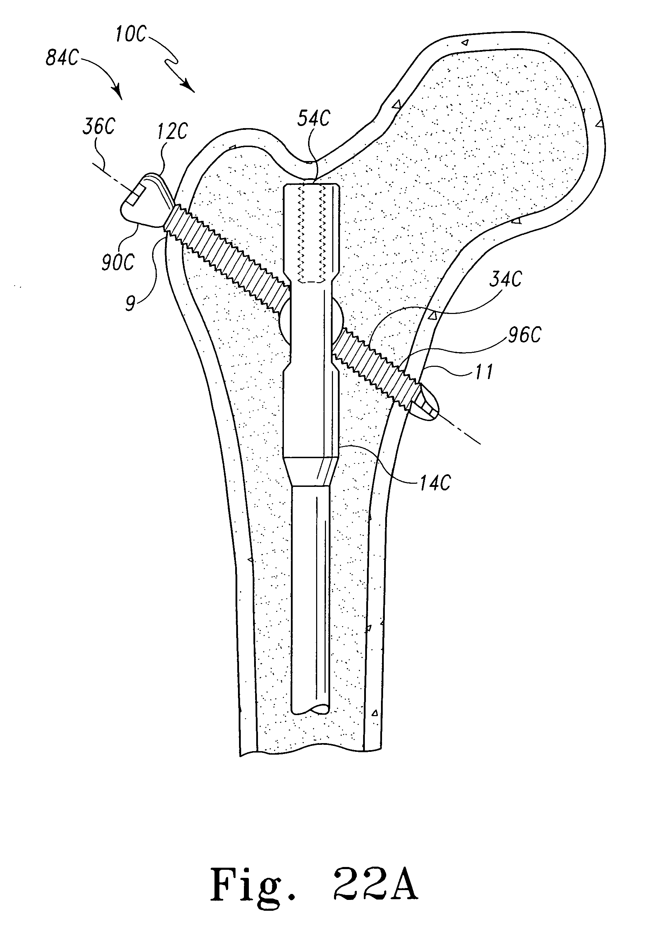

Referring now to FIG. 22, the nail assembly 10 is shown with the screw 12 to form the nail implant assembly 84. The nail implant assembly 84 is configured such that the nail implant assembly 84 is utilized to secure a greater trochanter to lesser trochanter fracture. The nail implant assembly 84 includes the nail 14, which together with the bushing 20 forms the nail assembly 10.

The bushing 20 is oriented such bushing centerline 36 extends from greater trochanter 9 to lesser trochanter 11. The bushing 20 of the nail assembly 10 is rotated into position such that the transverse centerline 36 of the bushing 20 is aligned from the greater trochanter 9 to the lesser trochanter 11 and then the locking plug 54 is used to secure the bushing 20 with respect to the nail 14.

The head 90 of the screw 12 rests against cortical bone 3 at the greater trochanter 9. The shank 34 of the screw 12 extends through bushing 20 and the threads 96 of the screw 12 extend into the femur 4 and may extend as shown in FIG. 22A through cortical bone 3 near the lesser trochanter 11 of the femur 4.

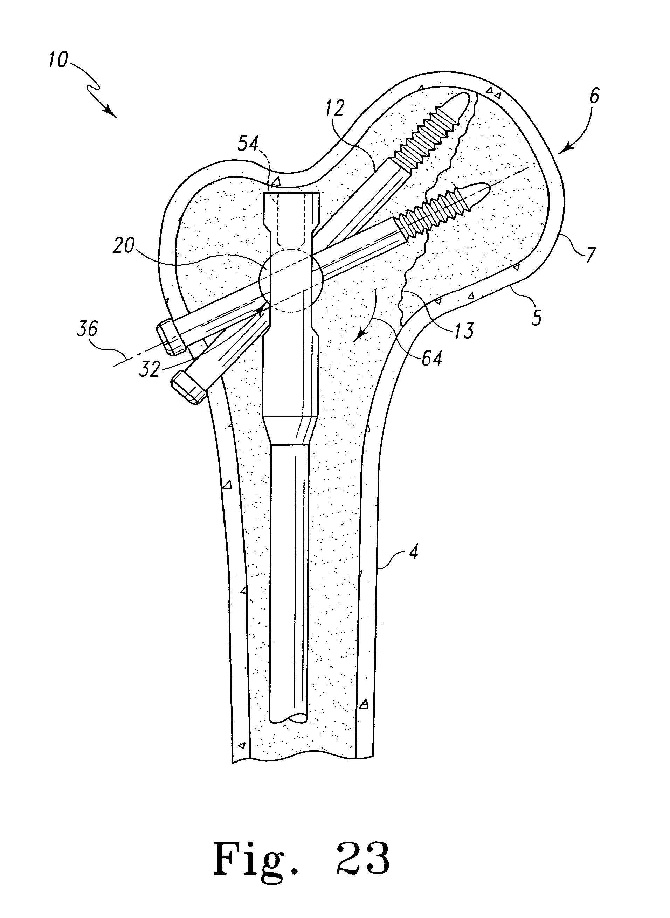

Referring now to FIG. 23, femur 4 is shown with fracture 13 extending through neck 5 of the femur 4. The fracture 13 extends transversely across the neck 5. As shown in FIG. 23, the bushing 20 of the nail assembly 10 may be rotated in the direction of arrow 64, such that the centerline 36 of the transverse opening 32 of bushing 20 is arranged such that the screw 12 when positioned in the bushing 20 of the nail assembly 10 intersects the fracture 13 such that the screw 12 serves to secure the head 7 to the remainder of the femur 4. As shown in FIG. 23, the longitudinal axis 36 of the opening 32 of the bushing 20 is aligned to properly secure the head 7 to the femur 4. The locking plug 54 is utilized to secure the bushing 20 to the nail 14.

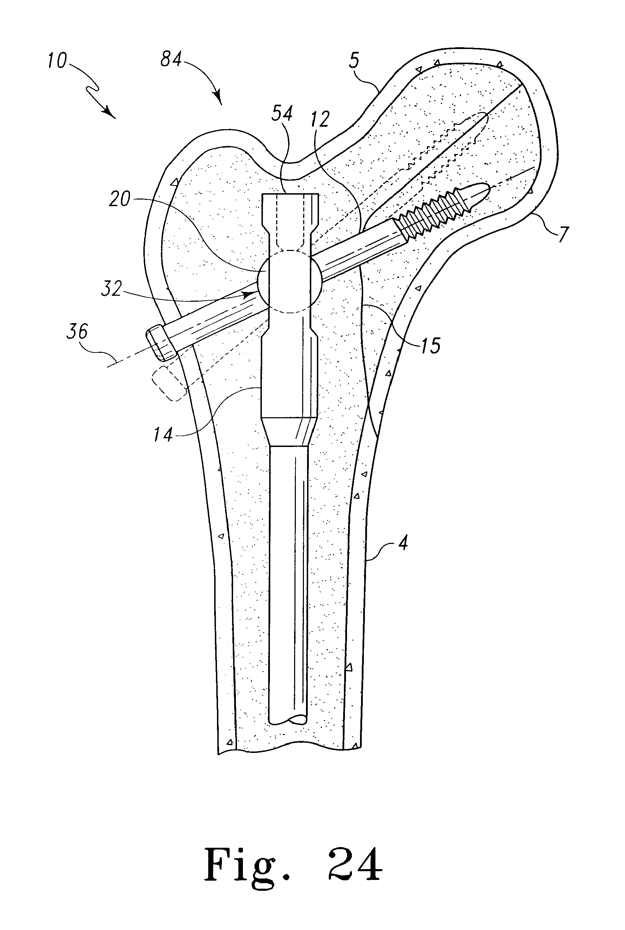

Referring now to FIG. 24, the nail implant assembly 84 of the present invention is shown utilized in the femur 4 in which a longitudinal fracture 15 extends from the body of the femur 4 through the neck 5 and into the head 7. It should be appreciated that the nail assembly 10, to properly secure the head 7 to the femur 4, may be oriented such that the screw 12 extends transversely through the fracture site 15.

The screw 12 as shown in FIG. 24, may be oriented in a direction different than the physical centerline of the neck 5 and head 7 such that the screw 12 may intersect the fracture 15 at a proper angle to properly secure the head 7 to the femur 4. Once the bushing 20 is oriented with the transverse axis 36 of the bushing 20 in a proper orientation, the locking plug 54 is secured against the bushing 20 to secure the locking plug 54 to the nail 14.

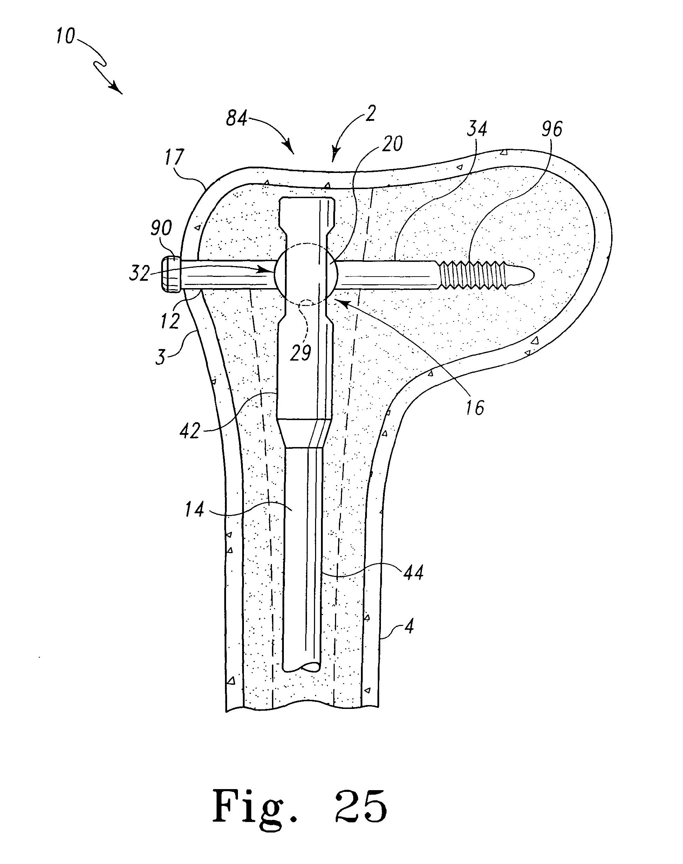

Referring now to FIG. 25, the nail implant assembly 84 of the present invention is shown inserted into the intramedullary canal 2 of the femur 4 from the distal condylar portion 17 of the femur 4. The nail implant assembly 84 includes the nail 14 and the bushing 20 forming the nail assembly 10. The nail assembly 10 is inserted through the distal condylar portion 17 of the femur 4 through the intramedullary canal 2. The screw 12 is inserted through the cortical bone 3 of the distal condylar portion 17 of the femur 4 and through the bushing opening 32 of the bushing 20 and into the femur 4. Such a nail implant assembly 84 implanted into the distal condylar portion 17 of the femur 4 is frequently called a retrograde nail.

While it should be appreciated that the nail implant assembly 84 of FIG. 1 may have the neck angle α that is secured interoperatively, it should be appreciated that the neck angle α may be preset on, for example, a bench in the operating room prior to implanting the nail assembly 10 into the patient.

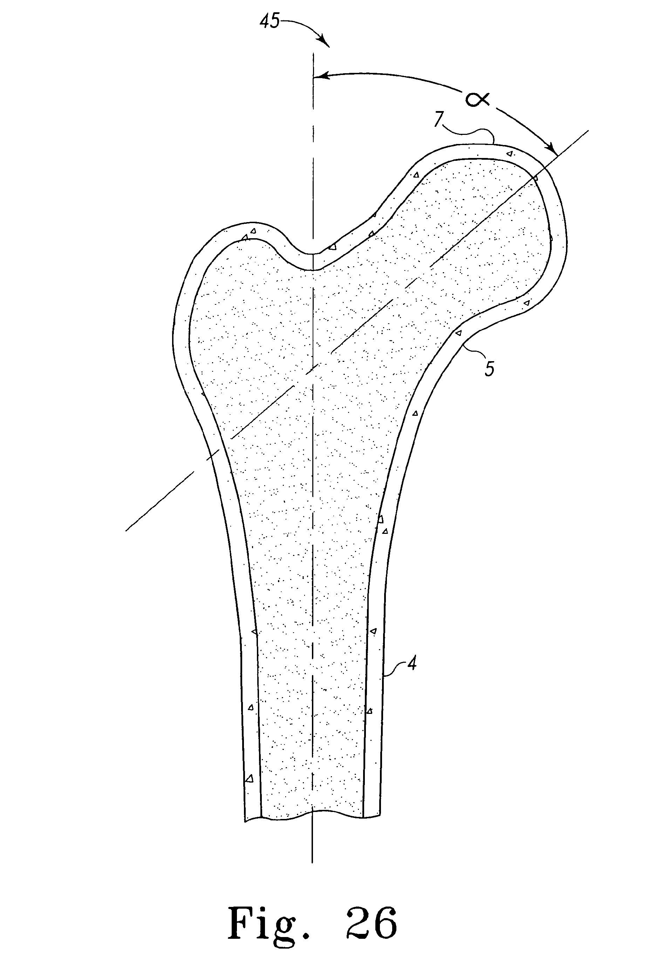

For example, and as shown in FIG. 26, an anterior/posterior view x-ray 45 of the femur 4 is shown. As shown in the x-ray 45, the neck 5 and head 7 of the femur 4 form an angle with the femur 4 defined as the neck angle α. The neck angle α may be determined from the x-ray 45.

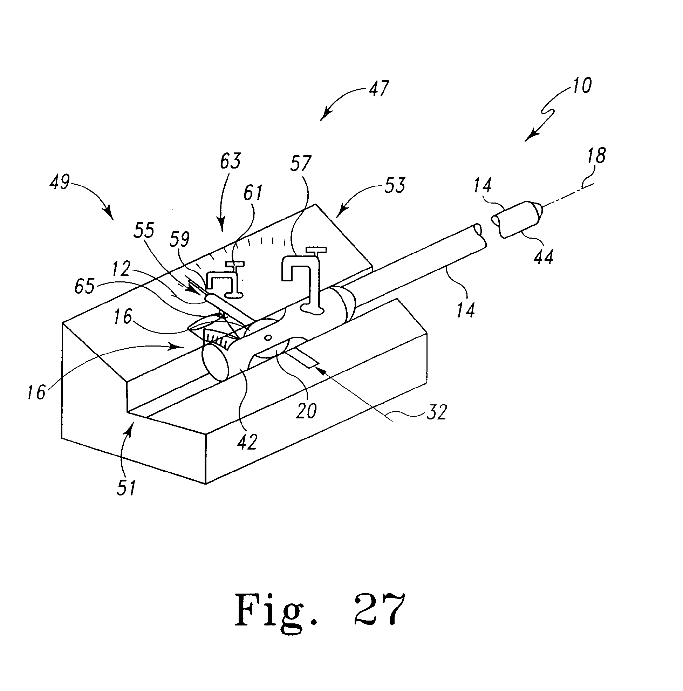

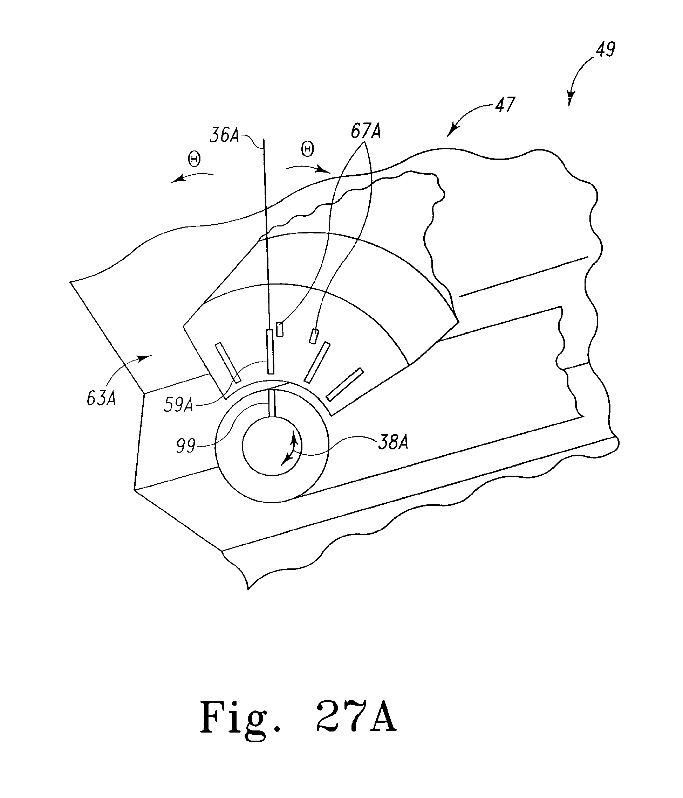

Referring now to FIG. 27, a device 47 for positioning the bushing 20 in the nail 14 is shown. The device 47 together with the nail assembly 10 form nail kit 49.

The device 47 may be in the form of a fixture for use with an intramedullary nail, for example, the nail assembly 10 of FIGS. 1-4. The nail assembly 10 includes a nail body, for example, nail 14 as shown in FIGS. 1-4. The nail assembly 10 further includes a screw feature, for example, bushing 20 for receiving a screw, for example screw 12 of FIG. 1, which can be orientated with respect to the nail 14. The fixture 47 is adapted to orient the bushing 20 with respect to the nail 14.

The fixture 47 includes a first portion 51 for cooperation with the nail 14. The fixture 47 further includes a second portion 53. The second portion 53 is capable of corresponding to one of a plurality of positions of the bushing 20 with respect to the nail 14. The fixture 47 further includes a screw feature cooperating feature 55, for example, as shown in FIG. 27, in the form of a fixture pin which slidably fits within opening 32 formed in bushing 20. The fixture pin may be in the form of, for example, the screw 12 or may be a separate component. The screw feature cooperating feature 55 is utilized with cooperating with the bushing 20 with the second portion 53.

The second portion 53 may, as shown in FIG. 27, be fixedly positioned with respect to the first portion 51. For example, and as shown in FIG. 27, the second portion 53 may be integral with the first portion 51. Alternatively, the position of the second portion 53 can be adjusted selectively with respect to the first portion. For example, the second portion 53 may be pivotally positioned with respect to the first portion 51. The pivoting position may correspond to the centre of the bushing 20.

The nail 14 may be positioned with respect to the first portion 51 by simple gravity causing the nail 14 to rest against the first portion 51. For example, and as shown in FIG. 27, the first portion 51 is in the form of a pair of planer surfaces that form a "V" to support the periphery of the nail 14. The planer surfaces that form the first portion 51 are positioned such that gravity holds the nail 14 against first portion 51. It should be appreciated that the nail 14 may be secured by a collet, a clamp, a biassing member or any device capable of positioning and retaining the nail 14 against the first portion 51.

The fixture 47 may further include a means for securing the nail 14 to the first portion 51 of the fixture. The means for securing may be in the form of, for example, a clamp 57, which may be mounted to the first portion 51 of the fixture 47.

The screw feature cooperating feature 55 may be any feature capable of cooperating with the bushing 20 to provide an indication of the orientation of the bushing 20. The screw feature cooperating feature 55 may be slidably fitted in the opening 32 formed in the bushing 20. The screw feature cooperating feature 55 may, in fact, simply be the screw 12 that is to be implanted in the patient. For simplicity and to avoid contamination of the screw 12 to be implanted, the screw feature cooperating feature 55 may be a separate component, for example, a cylindrical pin with which the second portion 53 cooperates. It should be appreciated that the screw feature cooperating feature 55 may include a tip or pointer 59 for cooperating with the second portion 53.

The fixture 47 may further include means for securing the screw feature cooperating feature 55 to the second portion 53 of the fixture 47. For example, the screw feature cooperating feature 55 may be in the form of the pin with the means for securing the pin 55 being in the form of a clamp 61 mounted to the second portion 53 and securing the pin 55 to the second portion 53.

The fixture 47 may further include a gage 63 for measuring the position of the screw feature 20 with respect to the nail 14. For example, the gage 63 may be in the form of a protractor 63 or a series of score marks positioned on the second portion 53 of the fixture 47. The protractor 63 may be generally planar and may overlay pointer 59 of the pin 55.

The fixture 47 may further include a preset feature 65 for providing a preset angular relationship of the transverse opening 32 with respect to the nail 14. For example, the preset feature 65 may be in the form of a spring biassed detent, which cooperates with the pin 55 to preset the angular relationship of the transverse opening 32 to a particular angular relationship α.

It should be appreciated that the fixture 47 may be adapted such that the first portion 51 and/or the screw feature cooperating feature 55 may be adapted to accommodate a plurality of intramedullary nails of different diameters, lengths and shapes. For example, and as shown in FIG. 27, the first portion 51 may be in the form of a pair of planar surfaces forming a "V" and therefore permitting the nail 14 to extend in opposed directions such that nails of 14 of various length may be utilized with the fixture 47. Further, the use of a first portion 51 with planar surfaces forming a "V" permits a variety of diameters of the nail 14.

Referring now to FIG. 27A, second protractor 63A of the device 47 of the nail kit 49 is shown. The second protractor 63A is somewhat similar to the first protractor 63 of FIG. 27. The second protractor 63A is used to rotatably orient the nail 14 in the wedge or "V" of the first portion 51 of the device 47. The second protractor 63A includes indicia 67A for cooperating with pointer 59A, which may extend from second cooperating feature 55A. The second cooperation feature 55A cooperates with an angular orientation feature 99 formed in the nail 14. The angular orientation feature 99 may be in the form of a set of notches that are in alignment linearly.

The nail 14 may be rotated in the direction of arrows 38A causing the pointer 59A to pass over different portions of the indicia 67A of the protractor 63A.

The pointer 59A may be moved, for example, a distance from centerline 36A, a distance of θ in both directions.

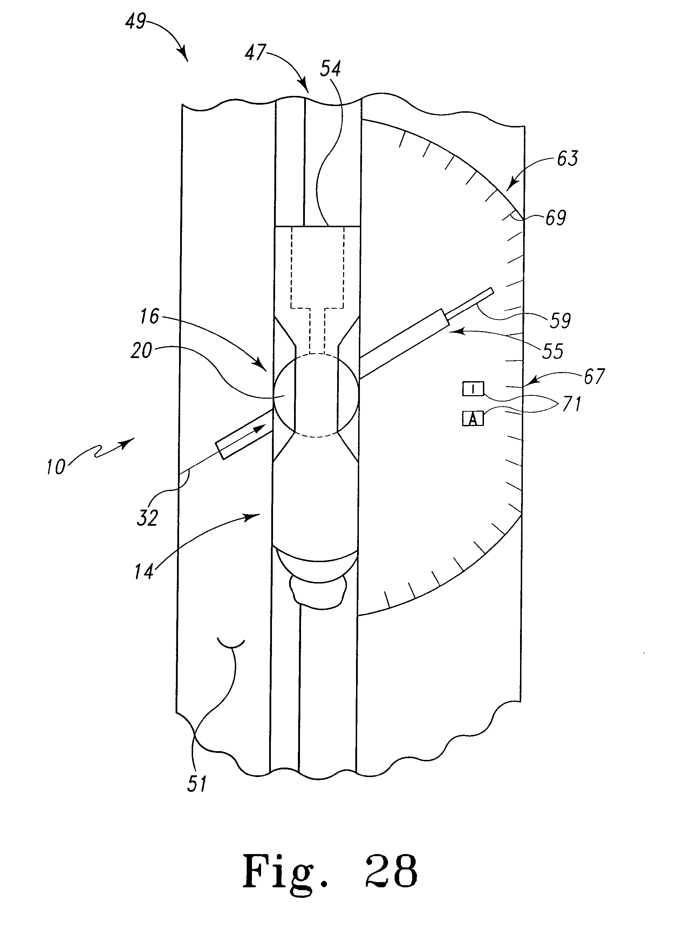

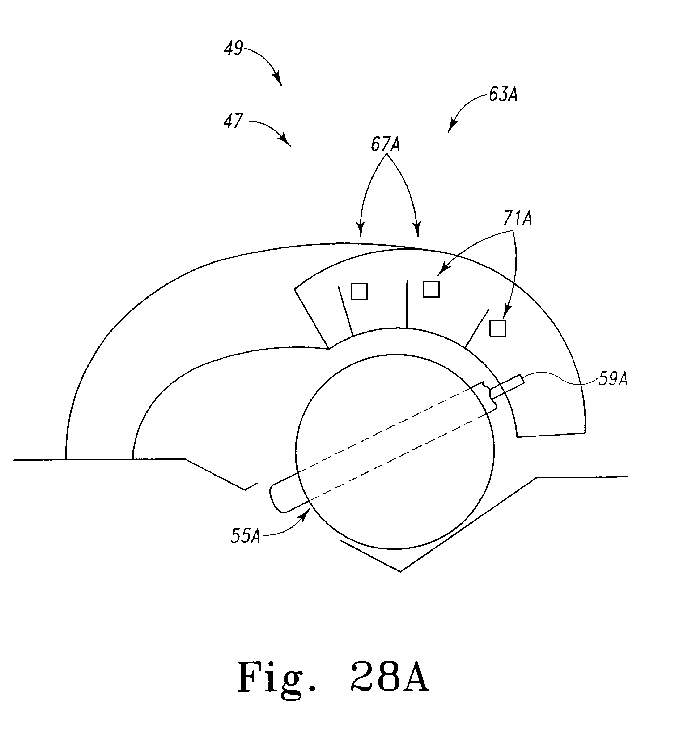

Referring now to FIG. 28, the pointer 59 is shown in position over the protractor 63. The protractor 63 may include indicia 67, which may be in the form of marks 69 and corresponding numbers or letters 71. The numbers and/or letters 71 may correspond to a particular angle or a particular desired angular position α of the bushing opening 32 with respect to the nail 14.

The corresponding feature or pin 55 may, as shown in FIG. 28, be rotated or aligned until the pointer 59 is aligned with the proper position indicated on the indicia 67 of the protractor 63. Once the pointer 59 is over the proper indicia 67 of the protractor 63, the locking pin 54 is rotated to secure the locking pin 54 against the bushing 20 so that the nail assembly 10 is properly oriented for implantation into the body. The pin 55 is then removed from the bushing opening 32 and the nail assembly 10 is ready for implantation.

Referring now to FIG. 28A, the device 47 of the nail kit 49 is shown with the protractor 63A shown in greater detail. The protractor 63A includes a cooperation feature 55A to which the pointer 59A extends. The pointer 59A is in alignment with indicia 67A formed on the protractor 63A. Characters 71A in the form of letters or numbers may be associated with the indicia 67A so that the position of the pointer 59A may be easily described.

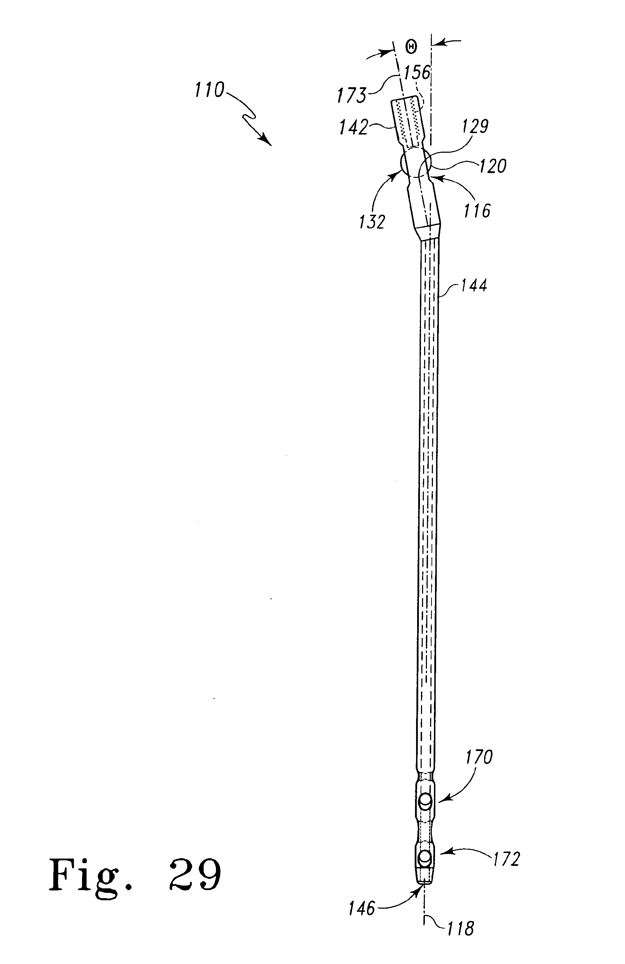

Referring now to FIG. 29, another embodiment of the present invention is shown as nail assembly 110. Nail assembly 110 is in the form of trochanteric nail assembly. The nail assembly 110 is similar to the nail assembly 10 of FIGS. 1-4, except that the trochanteric nail 114 of the nail assembly 110 is different than the nail 14 of the nail assembly 10 of FIGS. 1-4, in that the trochanteric nail 114 includes a proximal portion 142, which is skewed or not in alignment with distal portion 144 of the trochanteric nail 114.

The proximal portion 142 defines a proximal portion centerline 173 that forms an angle θ between centerline 118 of the distal portion 144. The angle θ is selected to facilitate the insertion of the trochanteric nail assembly 110 through the greater trochanter 9 of a femur 4.

As shown in FIG. 29, the trochanteric nail assembly 110 includes the trochanteric nail 114, which defines an aperture 116 in the proximal portion 142 of the nail 114. The aperture 116 receives a bushing 120, which defines a transverse opening 132 for receiving a screw 112. The bushing 120 is rotatably secured to the nail 114 by, for example, being contained between cradle 119 and locking pin 156. The nail 114 defines a longitudinal aperture 146 in alignment with longitudinal centerline 118. The nail 114 may, as shown in FIG. 28, include a first distal opening 170 and a second distal opening 172.

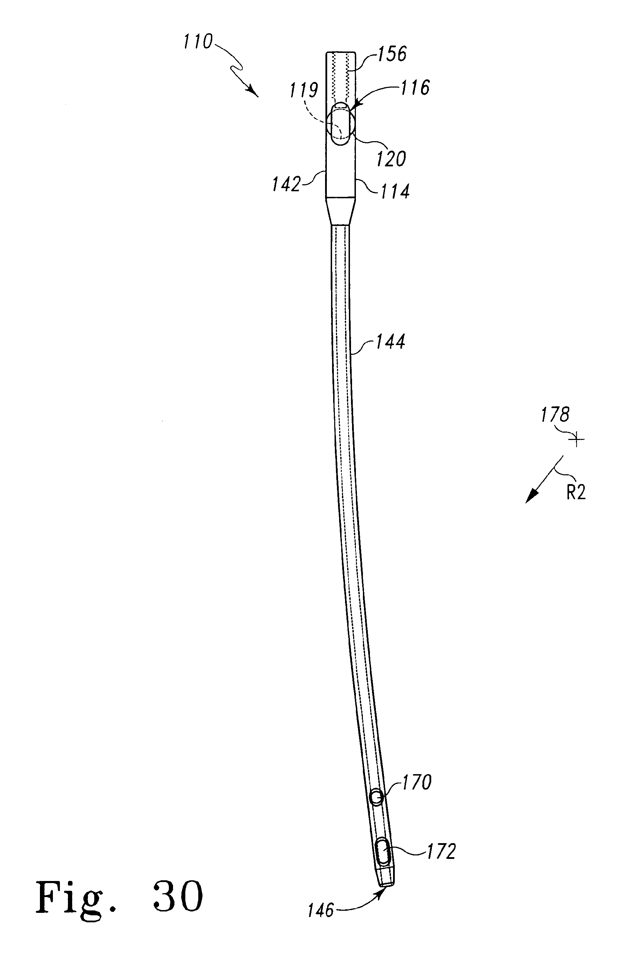

Referring now to FIG. 30, the distal portion 144 of the nail 114, may be straight or linear or may, as it is shown in FIG. 30, be arced or curved to conform with the arc or curve in the canal of a femur. As shown in FIG. 30, the distal portion 144 of the nail 114 is curved or forms an arc defined by radius R2 extending from origin 178.

While the nail of the present invention may be utilized for intramedullary nails for use with hip neck fracture, it should be appreciated that the nail of the present invention may be used elsewhere in long bone fractures.

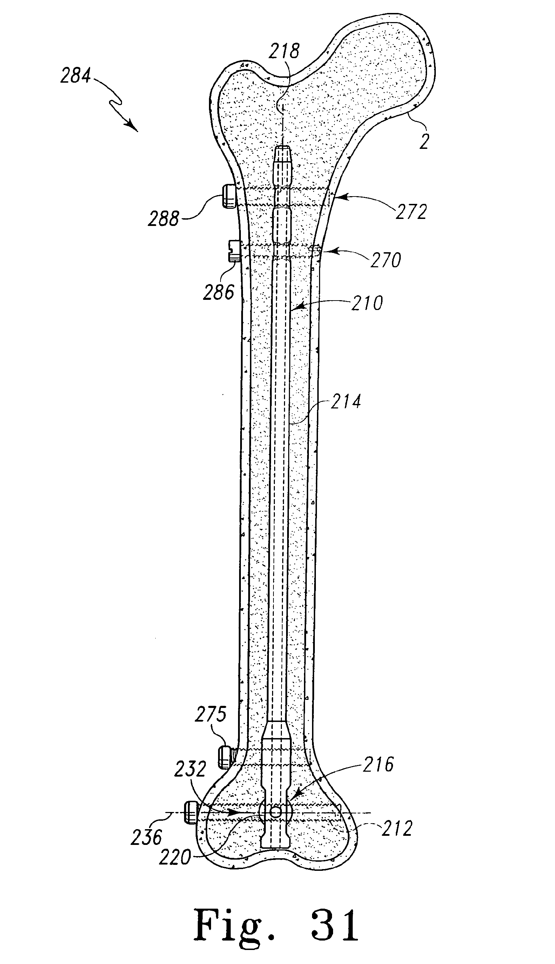

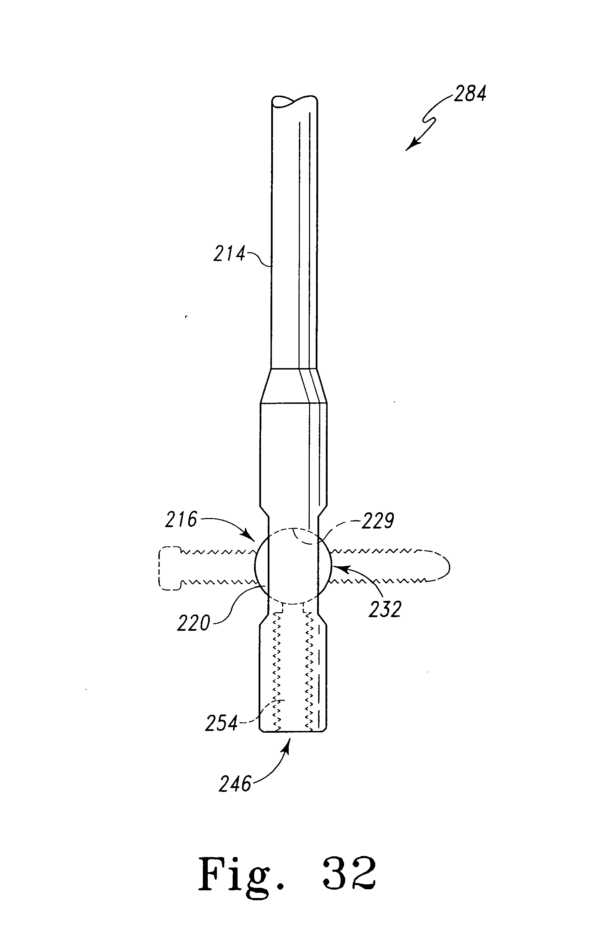

For example, and as shown in FIGS. 31 and 32, the nail of the present invention may be utilized for a retrograde femoral nail implant assembly 284 as shown in FIGS. 31 and 32.

Referring now to FIG. 31, the nail implant assembly 284 includes a nail assembly 210 to which screw 212 may be positioned in a plurality of angular positions. The nail implant assembly 284, as shown in FIG. 31, further includes a first proximal screw 286 that is slidably fitted to the first proximal opening 270 formed in the nail 214 of the nail assembly 210. The nail implant assembly 284 further includes a second proximal screw 288, which is slidably fitted to second proximal opening 272 formed in nail 214.

As shown in FIG. 31, the nail implant assembly 284 may further include a second distal screw 275, which is slidably fitted to nail 214. The nail implant assembly 284 includes the nail 214, which includes the distal opening 216 into which the bushing 220 is fitted. The bushing 220 includes an opening 232 into which the screw 212 is slidably fitted. The opening 232 of bushing 220 includes an axis 236, which may be adjusted as required with respect to the axis 218 of the nail 214.

Referring now to FIG. 32, the position of the bushing 220 can be adjusted and then locked by, for example, locking plug 254, which is threadably engaged with the nail 214. The bushing 220 may rotate between, for example, cradle 219 formed in the nail 214 and locking plug 254.

While the nail of the present invention may be particularly well suited for use with a femur, it should be appreciated that the nail of the present invention may be used with other long bones, for example, the tibia.

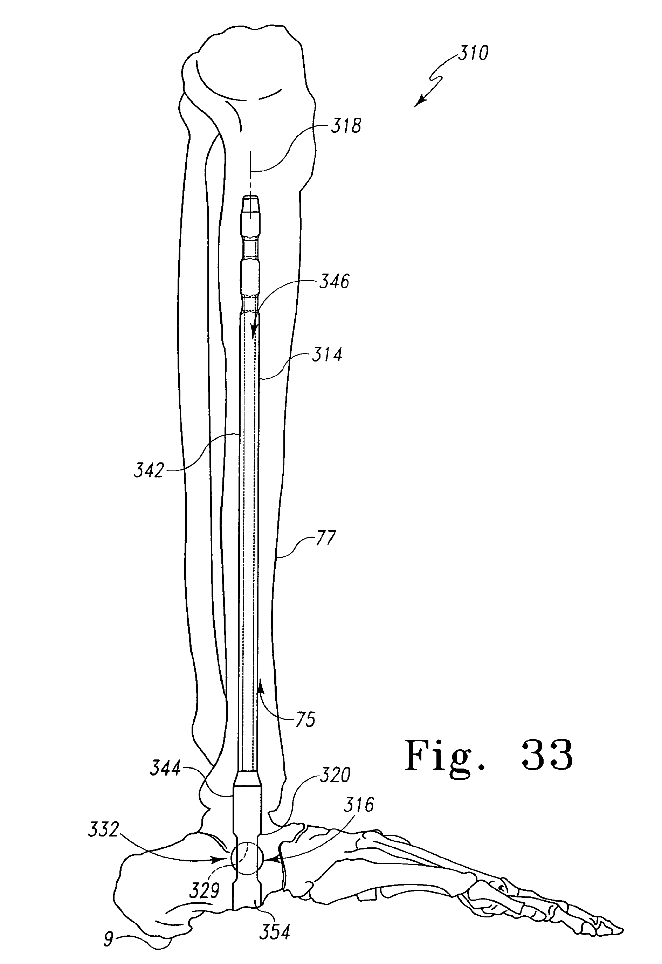

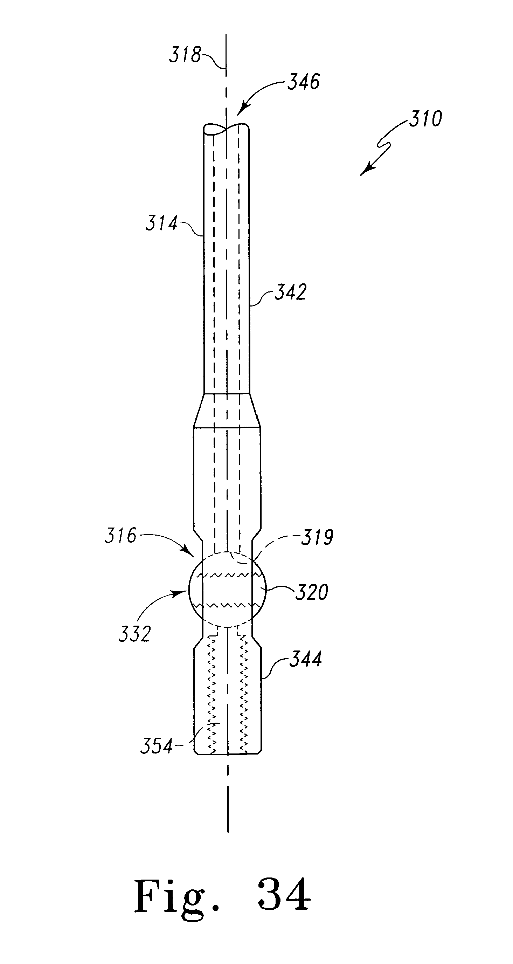

For example, and as shown in FIGS. 33 and 34, the nail of the present invention may be in the form of nail assembly 310. The nail assembly 310 as shown in FIGS. 33 and 34, may be in the form of a nail for use in a tibia. One such nail may be used, as shown in FIGS. 33 and 34, for use in nail fusion. The nail assembly 310, as shown in FIG. 33, may be inserted into the calcaneus 79 of the body and be inserted through the medullary canal 94 of the tibia 77.

The nail assembly 310 may include the nail 314. The nail 314 includes a transverse aperture 316 to which the bushing 320 is fitted. The bushing 320 is rotatably fitted to the nail 314 by, for example, being constrained between cradle 319 formed in nail 314 and locking plug 354. The nail assembly 310 includes a proximal portion 342, as well as, a distal portion 344 in which the aperture 316 is located. The distal portion 344 may further include the locking plug 354 to selectively lock the bushing 320 in place when opening 332 formed in the bushing 320 is properly positioned. The nail 314 may further include a longitudinal opening 346 extending along centerline 318 of the nail 314.

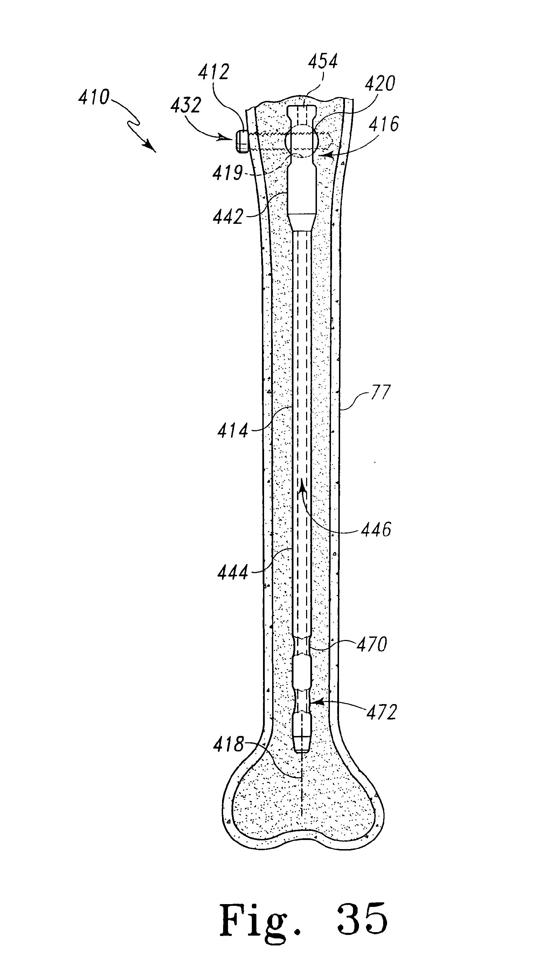

While the nail of the present invention may be utilized in a tibial nail with entry through the foot, it should be appreciated that the nail of the present invention may, as shown in FIG. 35, be in the form of, for example, nail assembly 410, which is inserted through the proximal portion of the tibia 77.

The nail assembly 410 includes a nail 414, which defines a transverse bushing opening 432 to which bushing 420 is fitted. The bushing 420 may be rotatably fitted in the bushing 420 by being constrained between cradle 419 formed in nail 414 and locking pin 454. The bushing 420 may include a bushing opening 432 for receiving screw 412 for forming the nail assembly 410. The nail 414 may further include a proximal portion 442 in which the bushing 420 is located as well as a distal portion 444, which may include distal openings 470 and 472. The nail 414 may be cannulated or include a longitudinal opening 446 formed along centerline 418 of the nail 414.

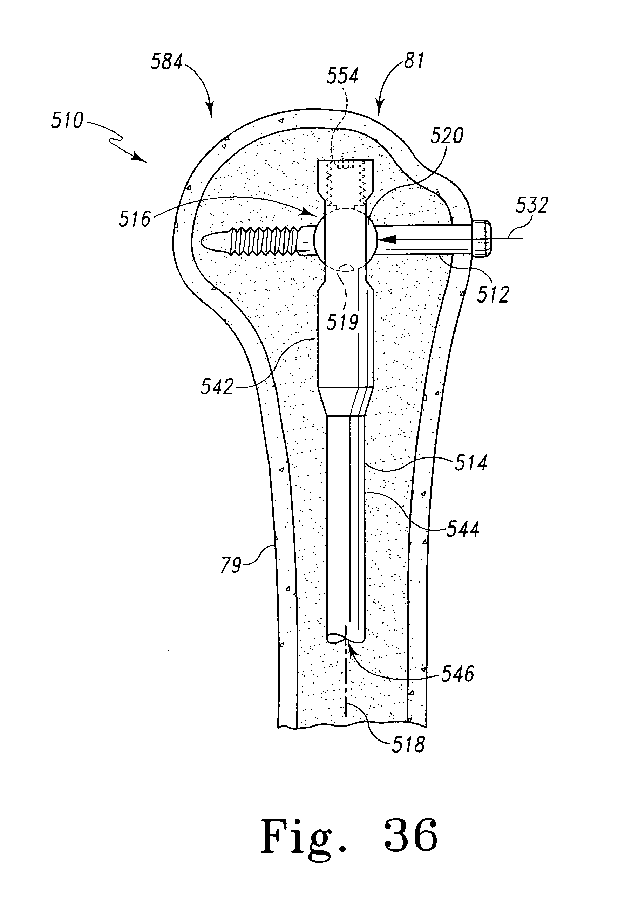

Referring now to FIG. 36, it should be appreciated that the nail assembly of the present invention may be used in other bones other than the long bones of the leg. For example, the nail of the present invention may be utilized in, for example, humerus 79.

The nail assembly 510 shown in FIG. 36 may include a nail 514, which is inserted proximally in intramedullary canal 81 of the long bone 79. The nail 514 may include an opening 516 into which a bushing 520 is rotatably fitted by, for example, being constrained between cradle 519 formed in nail 514 and locking plug 554 secured to the nail 514. The nail 514 and bushing 520 combine with the locking plug 554 to form the nail assembly 510.

The nail 514 may, as shown in FIG. 36, include a distal portion 544 defining a longitudinal centerline 518. The nail 514 may further include a proximal portion 542 extending from the distal portion 544. The proximal portion 542 includes the aperture 516.

The bushing 520 includes a bushing screw opening 532 through which a screw 512 may slidingly fit. The screw 512 and the nail assembly 510 combine to form the nail implant assembly 584. Nail 514 may include a longitudinal opening 546 extending concentric with longitudinal centerline 518 of the nail 514.

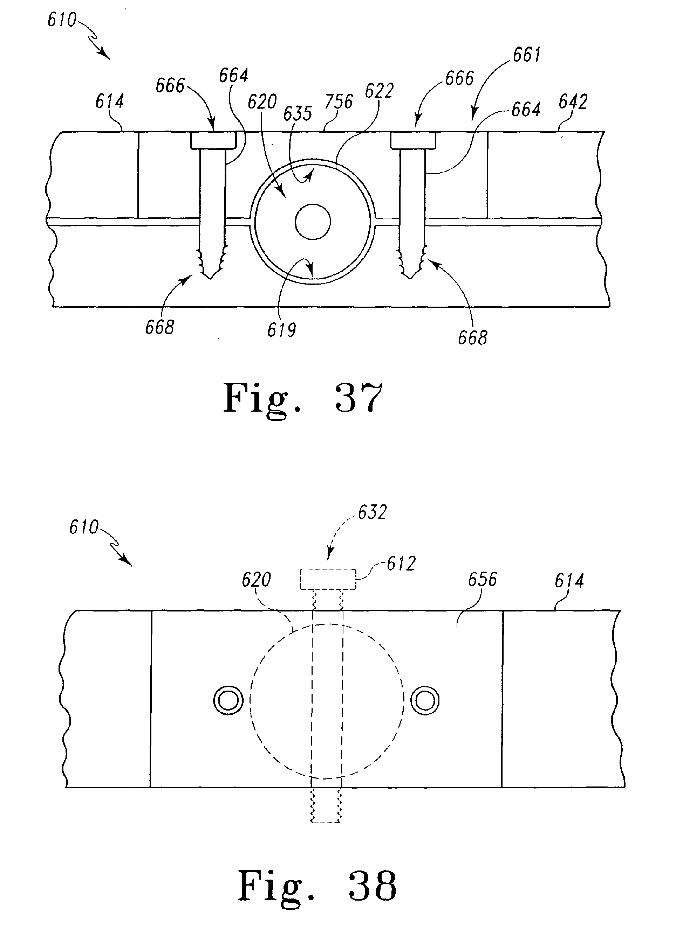

Referring now to FIG. 37, yet another embodiment of the present invention is shown as nail assembly 610. The nail assembly 610 includes a nail 614. The nail 614 includes a proximal portion 642, which defines a pocket 661 for receiving a plug 756. The nail 614 defines a cradle 619 for partially receiving bushing 620. The cradle 619 is, as shown in FIG. 37, concave and as shown in FIG. 37, generally hemispherical. The plug 756 includes a concave surface 635, which is similarly generally hemispherical and also mates with the periphery 622 of the bushing 620. The plug 756 is secured to the nail 614 in any suitable fashion. For example, and as shown in FIG. 37, the plug 756 includes transverse opening 666, which receive screws 664. The screws 664 mate with threaded pockets 668 formed in the nail 610.

Referring now to FIG. 38, the nail assembly 610 is shown with the nail 614 as well as with the plug 756. As shown in FIG. 38, the nail assembly 610 may further include a screw 612 slidingly fitted into the opening 632 formed in the bushing 620 contained by the plug 756.

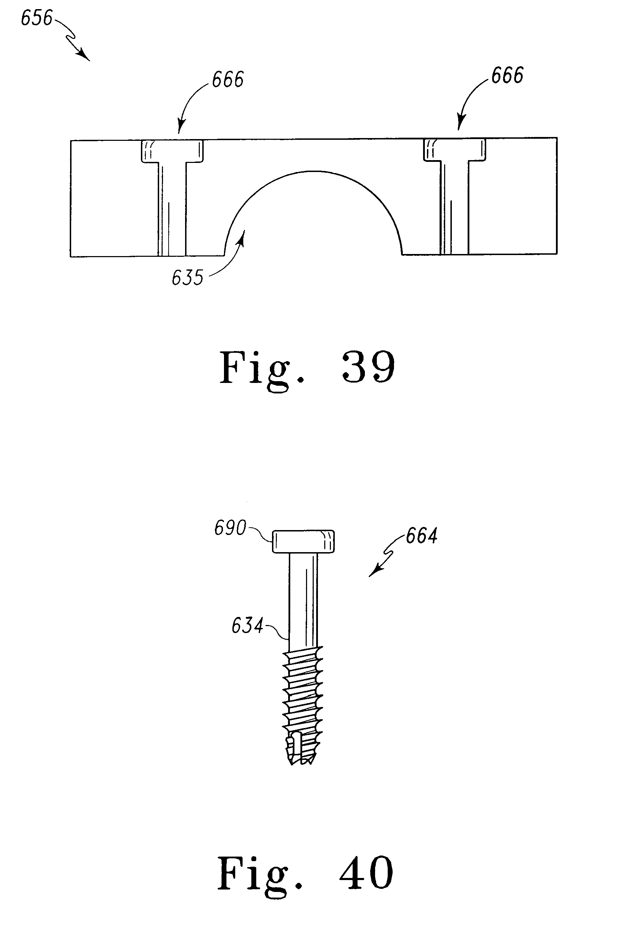

Referring now to FIG. 39, the plug 756 is shown in greater detail. The plug 756 includes the transverse openings 666 for receiving the screws 664 as well as concave surface 635 for receiving the bushing 620.

Referring now to FIG. 40, the screw 664 is shown in greater detail. The screw 664 may include head 690 as well as threaded shank 634.

While the present invention is shown in FIGS 1-40 provides for a bushing that rotates within an intramedullary nail, it should be appreciated that the present invention may be in the form of a nail with a bushing that translates as well as rotates. It should be appreciated that the bushing 720 may have a flat or protrusion (not shown) that cooperates with a flat or recess (not shown) on the nail 714 to prevent rotation of the bushing 720. In such cases, the bushing will only rotate about one axis.

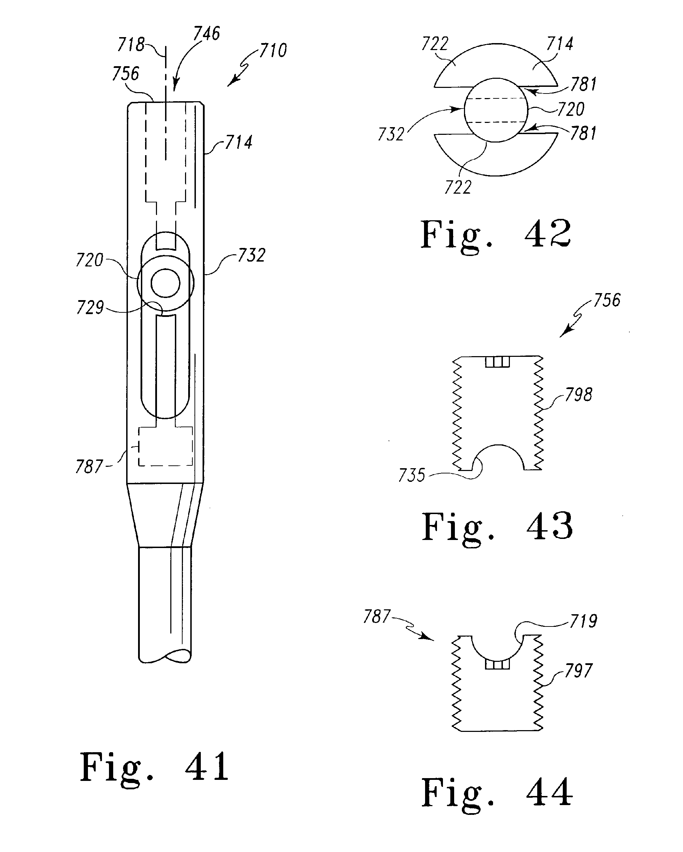

For example, and as shown in FIGS. 41-44, another embodiment of the present invention is shown as nail assembly 710. The nail assembly 710 includes a nail 714, which cooperates with a bushing 720. The bushing 720 rotates and translates along longitudinal axis 718 of the nail 714.

The bushing 720 may translate and rotate any suitable fashion along the longitudinal axis 718. For example, and as shown in FIG. 42, the bushing 720 may translate and rotate along a cylindrical opening 746 formed in the nail 714. The bushing 720 includes periphery 722, which mates with arcuate portions 781 formed in the nail 714. The arcuate portion 781 may be part of the cylindrical opening 746.

Referring again to FIG. 41, the bushing 720 may be positioned in the nail 714 and locked against movement in any suitable manner. For example, and as shown in FIG. 43, the nail assembly 710 may further include a distal locking plug 756 threadably engaged to the nail 714 for positioning one end of the bushing 720. Opposed to the distal locking bushing 756, a proximal locking bushing 787 may be threadably engaged with the nail 714 and be positionable against the bushing 720 to lock the bushing 720 between the distal locking plug 756 and the proximal locking plug 787.

As shown in FIG. 41, an opening 732 is formed in bushing 720 to receive the screw. If the nail assembly 710 is used as a nail for use with a screw in a femoral neck, the opening 732 may be oriented in the angle proper to have the screw enter into the neck and into the head of the femur. If the screw is used differently, the opening 732 may be, for example, normal or perpendicular to longitudinal axis 718.

Referring now to FIG. 43, the distal plug 756 is shown in greater detail. The distal plug 756 includes external threads 798 for thread engagement with the nail 714, as well as a concave surface 735 for receiving external periphery 722 of the bushing 720 (see FIGS. 41 and 42).

Referring now to FIG. 44, proximal bushing plug 787 is shown in greater detail. The proximal bushing plug 787 has a generally cylindrical shape and includes external threads 797 for engaging with the nail 714. The bushing plug 787 may further include a cradle 719 for cooperation with external periphery 722 of the bushing 720.

While the present invention may be utilized for a nail having a unitary screw, which may be positioned at a plurality of angles, it should be appreciated that the present invention may be used with a nail having two screws for positioning at a variety of angles.

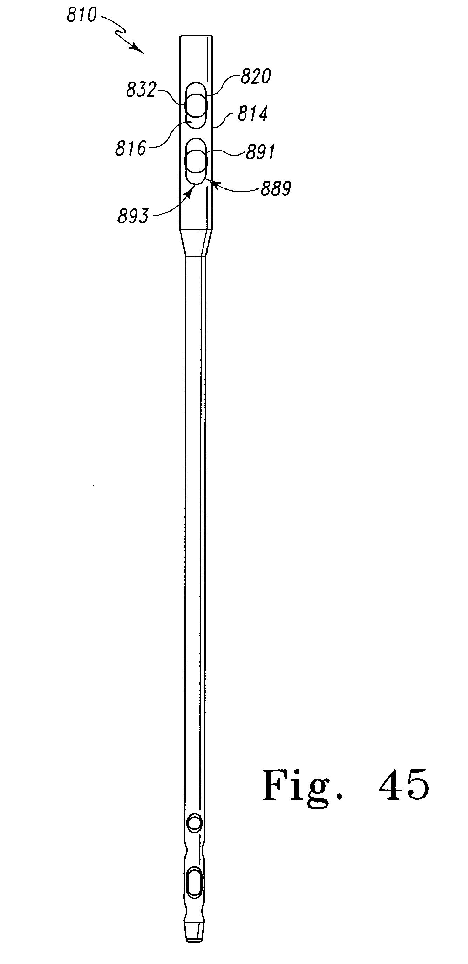

For example, and according to the present invention, and referring now to FIG. 45, nail assembly 810 is shown. The nail assembly 810 includes a nail 814 having a first opening 816 and a second spaced apart nail aperture 889. A first bushing 820 is rotatably positioned in first nail opening 816. A first bushing opening 832 is formed in the first bushing 820 and is adapted for receiving a first screw (not shown). The second nail aperture 889 is adapted for receiving a second bushing 891. The second bushing 891 is rotatably positioned in the nail 814 and includes a second bushing opening 893 formed in the second bushing 891.

It should be appreciated that the second bushing opening 893 is adapted for receiving a second screw while the first bushing opening 832 is adapted for receiving a first screw. It should be appreciated that the first screw and second screw may be oriented in a similar direction. For example, both the first screw and the second screw may be positioned into the neck and head of a femur. The second screw may be in the form of an anti-rotation screw to provide for proper securement of a head and/or a neck of a femur to a bone shaft after a femur-neck fracture.

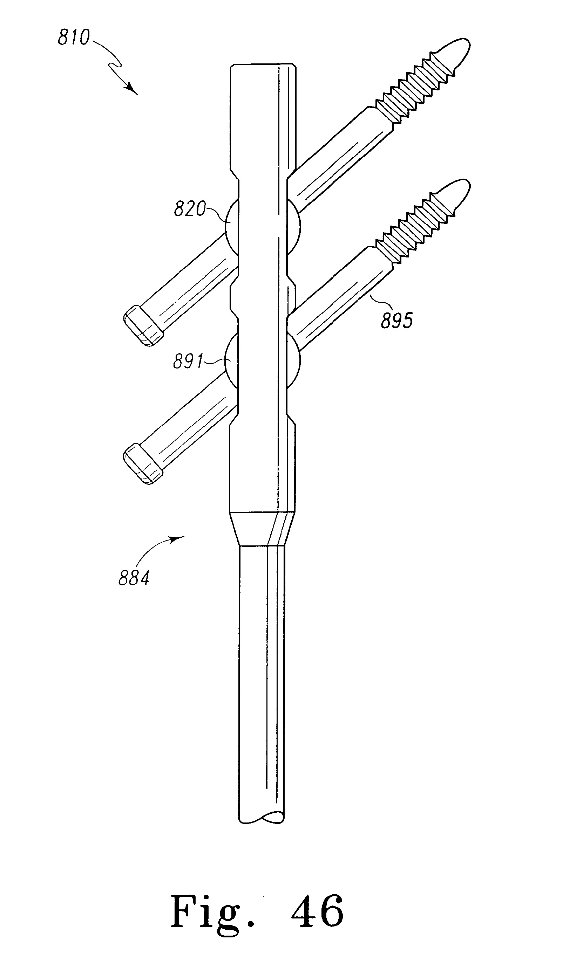

Referring now to FIG. 46, nail implant assembly 884 according to the present invention is shown. The nail implant assembly 884 includes the nail assembly 810 including the nail 814 as well as first bushing 820 and second bushing 891. A first screw 812 is slidably fitted into the first bushing 820, while a second screw 895 is slidably fitted through the second bushing 891. First screw 812 and the second screw 895 extend into neck 5 and head 7 of the femur 4.

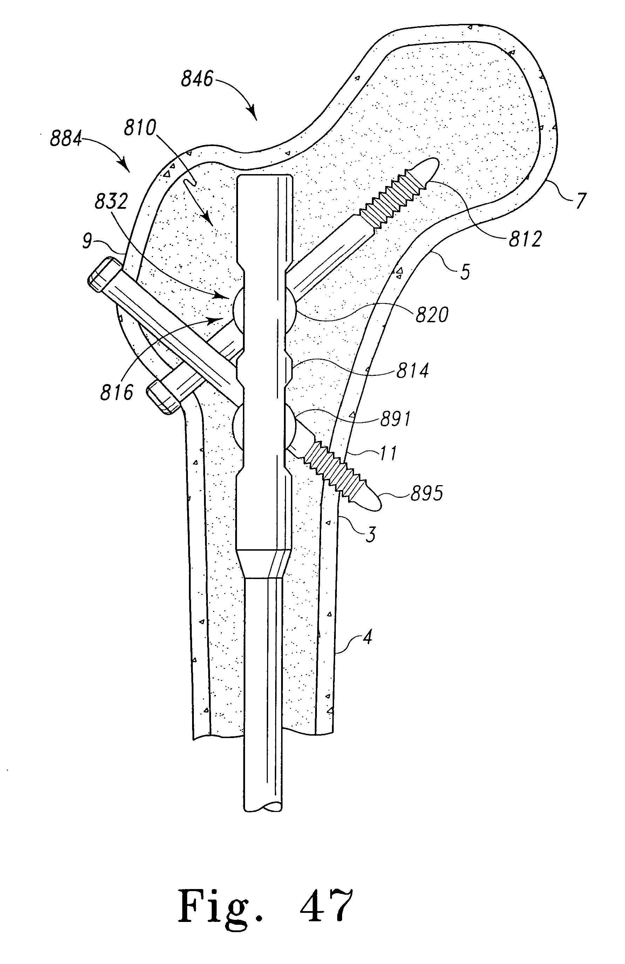

Referring now to FIG. 47, another use for the nail implant assembly 884 of FIG. 46 is shown in greater detail. The nail implant assembly 884 includes the nail assembly 810 as well as a first screw 812 and a second screw 895. The nail 814 is fitted into medullary canal of the femur 4 and the first screw 812 is slidably fitted through opening 832 formed in bushing 820 fitted into aperture 816 of the nail 814. The screw 812 as is shown in FIG. 47 may extend into the cancellous bone in the neck and head of the femur 4.

As shown in FIG. 47, the nail implant assembly 884 has the ability by having two spherical bushings to present a second screw in this case, a cortical screw 895 in a plane skewed from the first screw 812 permitting the second screw 895 to pass near the first screw 812 and permit the nail 864 to accommodate both a neck fracture and a greater trochanter to lesser trochanter fracture.

The nail assembly 864 further includes the second screw 895, which is shown in FIG. 47, is in the form of a cortical screw. The cortical screw 895 extends from the greater trochanter 9 to the lesser trochanter 11. The cortical screw 895 is used to having the distal portion of the screw 895 extend into cortical bone 3.

The cortical screw 895 may, as shown in FIG. 47, extend through greater trochanter 9 and into the femur 4. The cortical screw 895 enters through the second bushing 891 and into cortical wall 3 of the lesser trochanter 11, thereby rigidly securing the cortical screw 895 to a opposed cortices at the greater trochanter 9 and lesser trochanter 11.

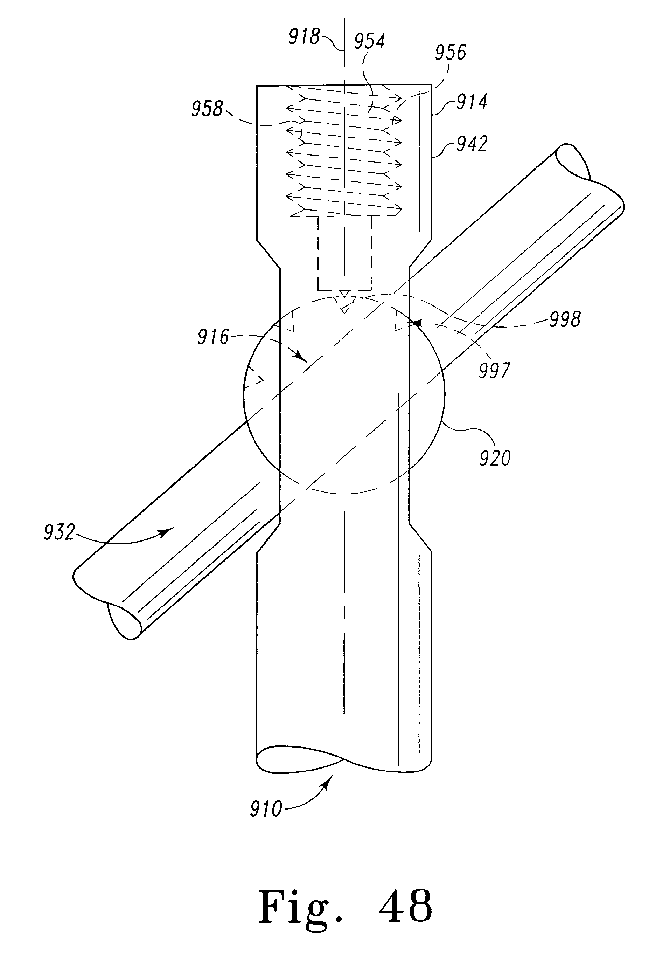

According to the present invention, and referring now to FIG. 48, yet another embodiment of the present invention is shown as nail assembly 910. The nail assembly 910 is similar to the nail assembly 10 of FIGS. 1-4, but further includes a provision for pre-selecting certain angular orientations of the screw opening in the proximal portion of the nail.

For example, and as shown in FIG. 47, the nail assembly 910 includes a nail 914 having a proximal portion 942. The proximal portion 942 defines an aperture 916 for receiving a bushing 920. The bushing 920 defines an opening 932 through the bushing 920 for receiving a screw (not shown). The bushing 920 may be preselected at a plurality of different angular orientations by the use of a preselection or locking feature.

For example, and as shown in FIG. 47, the bushing 920 may include a plurality of spaced apart indentations 997, which cooperate with a tip 998 formed in locking plug 954. The locking plate 954 includes external threads 956, which cooperate with internal threads 958 formed in the nail 914. The tip 998 in the indentations 997 cooperate with each other to provide for certain preset locking angles of the opening 932 with respect to longitudinal axis 918 of the nail 914.

Referring now to FIG. 48, the nail assembly 910 includes the nail 914 as well as the bushing 920. The bushing 920 includes indentations 997, which as shown in FIG. 48, extend in the medial/lateral plane as well as the anterior/posterior plane. This provides a selected position that accommodates both movement in the medial/lateral plane and the anterior/posterior plane. The indentations 997 cooperate with tips 998 formed in the nail 914. The bushing 920 includes a central hole or opening 932 for receiving screw 912. The nail 914 may include a longitudinal opening 946 extending axially through the nail 914.

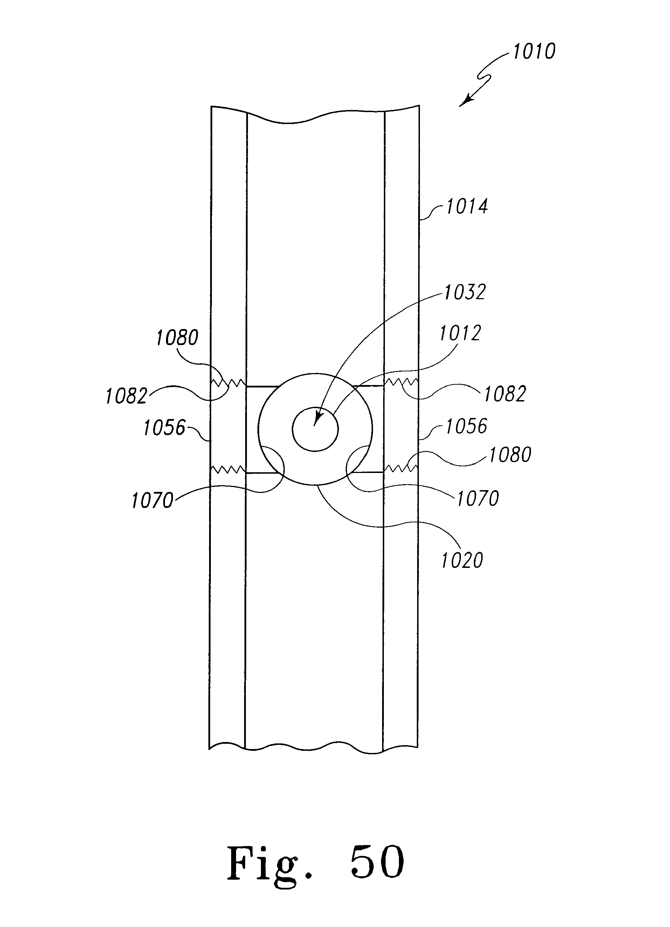

Referring now to FIGS. 50, 51 and 52, yet another embodiment of the present invention is shown as nail assembly 1010. The nail assembly 1010 includes a nail 1014 as well as a bushing 1020. The bushing 1020 includes a central opening 1032 for receiving screw 1012. The bushing 1020 is contained within the nail 1014 in a different manner. For example, and as shown in FIG. 50, the nail assembly 1010 further includes opposed plugs 1056, which are threadably engaged to the nail 1014. For example, and as shown in FIG. 50, the plugs 1056 include external threads 1080, which engage with internal threads 1082 formed in the nail 1014. Plugs 1056 include a plug concave surface 1070, which may have a generally hemispherical shape to mate with external periphery 1022 of the bushing 1020.

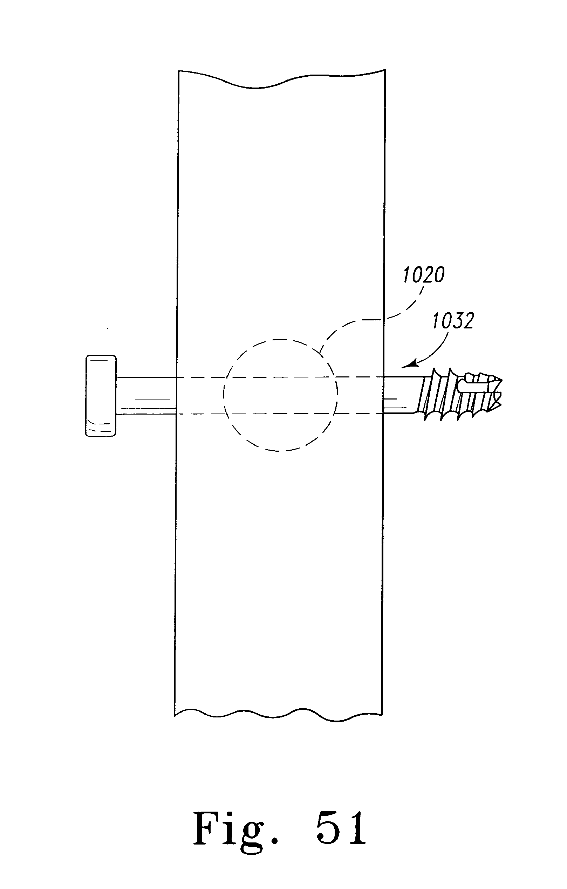

Referring now to FIG. 51, nail implant assembly 1084 is shown. The nail implant assembly 1084 includes the nail 1010 of FIG. 50 as well as screw 1012, which is slidably fitted into opening 1032 formed in bushing 1020 of the nail 1014. The screw 1012 may include a head 1090 for engagement with the bone, for example, femur 4.

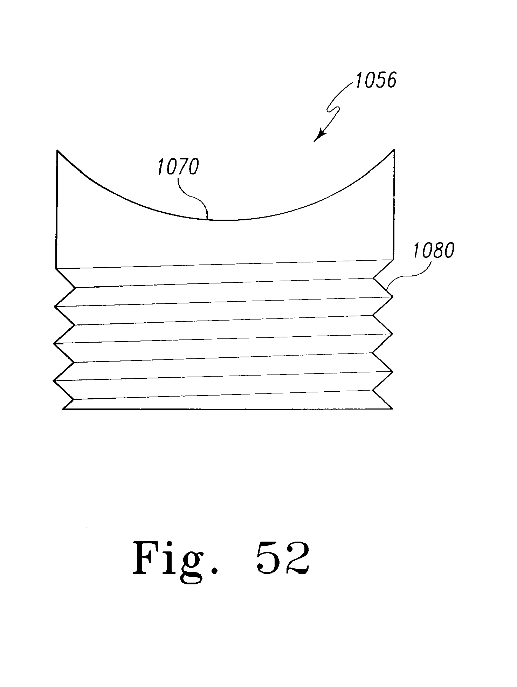

Referring now to FIG. 52, the plug 1056 is shown in greater detail. The plug 1056 includes the plug concave surface 1070 as well as external threads 1080.

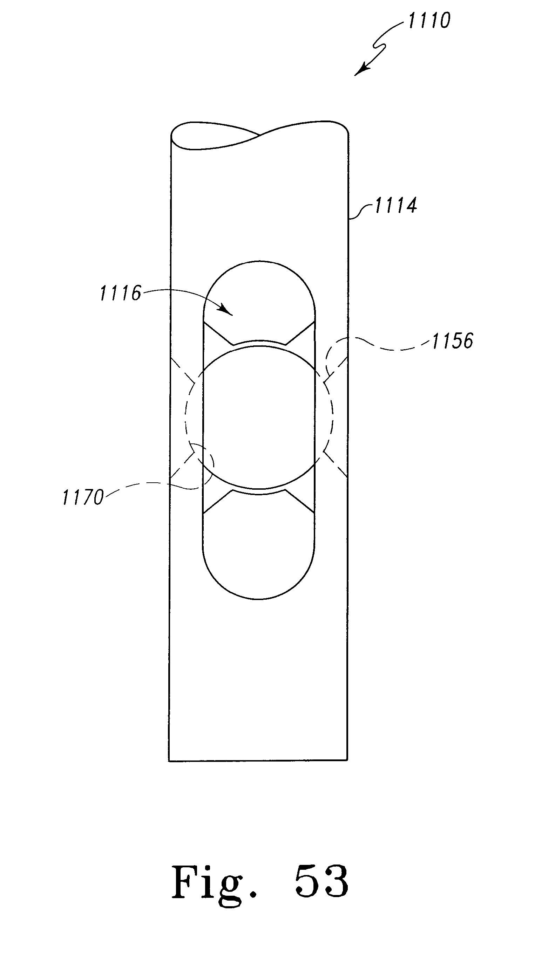

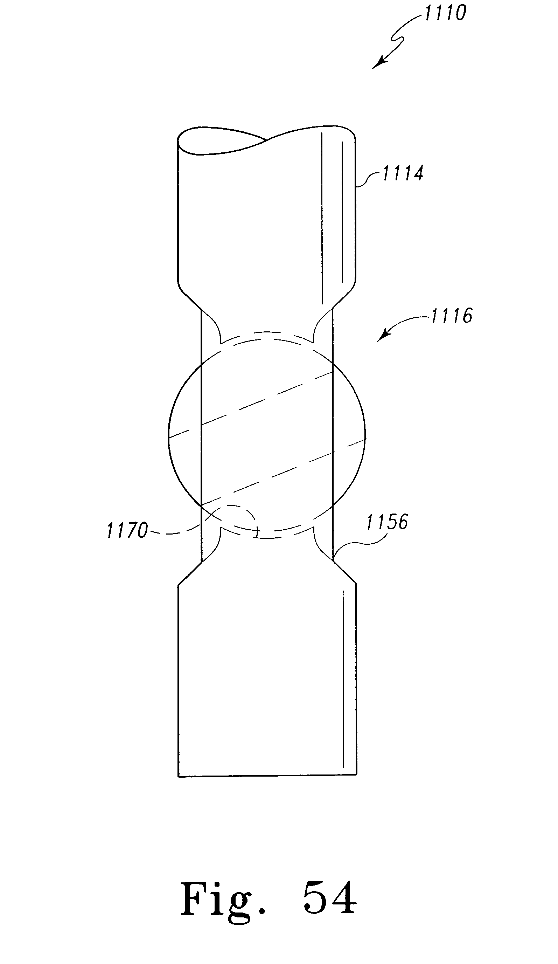

Referring now to FIGS. 53 and 54, yet another embodiment of the present invention is shown as nail assembly 1110. The nail assembly 1110 as shown in FIG. 53, includes nail 1014. The nail 1014 defines aperture 1016 for receiving bushing 1120. The bushing 1120 is snap fitted into the aperture 1116 by any suitable means.

For example, and as shown in FIG. 53, the nail 1114 includes lips or protrusions 1170, which extend into the aperture 1116. The lips 1170 require a deflection of the lips 1170 when inserting the bushing 1120 into the aperture 1116.

Referring now to FIG. 54, nail implant assembly 1184 is shown. The nail implant assembly 1184 includes the nail assembly 1110 as well as screw 1112. The screw 1112 is slidably fitted into opening 1132 formed in the busing 1120.

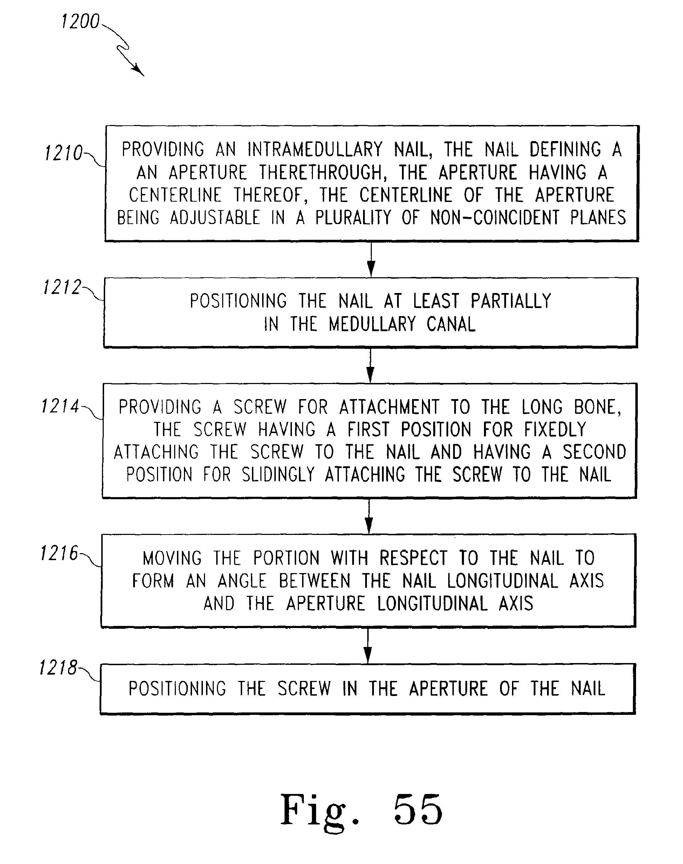

Referring now to FIG. 55, yet another embodiment of the present invention is shown as surgical procedure or surgical method 1200. The method 1200 includes a first step 1210 of providing an intramedullary nail. The nail defines an aperture through the nail. The aperture has a centerline. The centerline of the aperture is adjustable in a plurality of non-coincident planes. The method 1200 includes a second step 1212 of positioning the nail at least partially in the medullary canal.

The method 1200 includes a third step 1214 of providing a screw for attachment to the long bone. The screw has a first position for fixedly attaching the screw to the nail, and has a second position for slidably attaching the screw to the nail. The method 1200 further includes a fourth step 1216 of moving the portion with respect to the nail to form an angle between the nail longitudinal axis and the aperture longitudinal axis. The method 1200 further includes a fifth step 1218 of positioning the screw in the aperture of the nail.

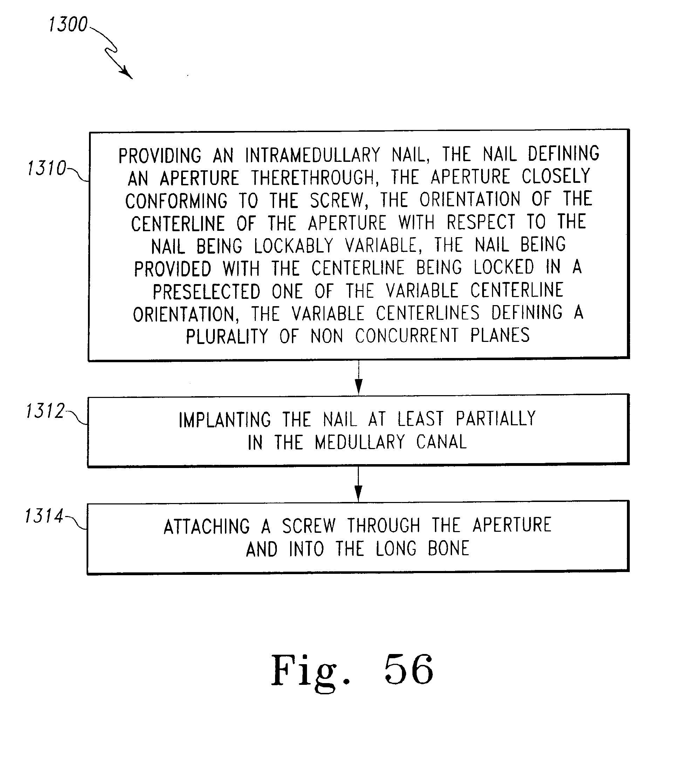

Referring now to FIG. 56, yet another embodiment of the present invention is shown as a method or surgical procedure for performing trauma surgery on a long bone. The method includes the steps of providing a screw for attachment to the long bone. The method includes a first step 1310 of providing a screw for attachment to the long bone. The method 1300 further includes a second step 1312 of providing an intramedullary nail. The nail defines an aperture through the nail. The aperture closely conforms to the screw.

The orientation of the centerline of the aperture with respect to the nail can be varied and locked. The nail is provided with a centerline that may be locked in a preselected one of variable centerline orientations. The variable centerlines define a plurality of non-concurrent planes. The method 1300 further includes a third step 1314 of implanting the nail at least partially in the medullary canal. The method 1300 further includes a fourth step 1316 of attaching a screw through the aperture and into the long bone.

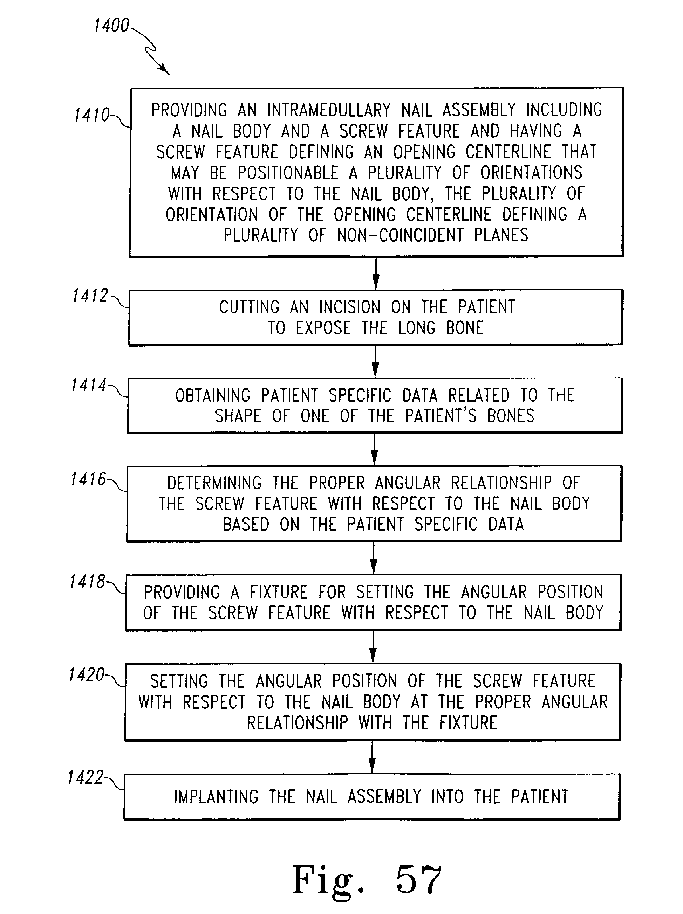

Referring now to FIG. 57, yet another embodiment of the present invention is shown as method of performing trauma surgery on a long bone of a patient or method 1400. The method 1400 includes a first step 1410 of providing an intramedullary nail assembly, including a nail body and a screw feature. The nail includes a screw feature defining an opening centerline whose orientation with respect to the nail can be adjusted. The plurality of orientations of the opening centerline define a plurality of non-incident planes.

The method 1400 further includes a second step 1412 of providing an incision on the patient to expose the long bone. The method 1400 further includes a third step 1414 of obtaining patient specific data related to the shape of one of the patient's bones. The method 1400 further includes a fourth step 1416 of determining the proper angular relationship of the screw feature with respect to the nail body based on the patient specific data.

The method 1400 further includes a fifth step 1418 of providing a fixture for setting the angular position of the screw with respect to the nail body. The method 1400 further includes a sixth step 1420 of setting the angular position of the screw feature with respect to the nail body at the proper angular relationship with the fixture. The method 1400 further includes a seventh step 1422 of implanting the nail assembly into the patient.

## Claims

1. An intramedullary nail for use with a bushing and a screw in a medullary canal of a long bone, said assembly comprising:
a body for positioning at least partially in the medullary canal, said body defining a body aperture therethrough, said body further defining a longitudinal axis thereof; and
an orientation feature operably connected to said body, said orientation feature adapted to support the bushing so that the bushing may be moveably positionable with respect to said body so that the bushing may receive the screw in a plurality of angular orientations with respect to the longitudinal axis of said body, said plurality of angular orientations defining a plurality of non-coincident planes.

2. The nail as in claim 1, wherein said orientation feature provides for translatable positioning of the bushing with respect to said body.

3. The nail as in claim 1, wherein said orientation feature provides for rotatable positioning of the bushing with respect to said body.

4. The nail as in claim 1, in which the first mentioned bushing defines a first bushing opening therethrough for receiving at least a portion of the screw; and in which the nail includes a second orientation feature for cooperation with a second bushing, the second bushing defining a second opening therethrough for receiving at least a portion of the screw, the first mentioned bushing opening and the second bushing opening capable of having different angular orientations with respect to the longitudinal axis of said body.

5. The nail as in claim 1, wherein at least a portion of said body is cannulated along the longitudinal axis.

6. The nail as in claim 1, wherein at least a portion of said body defines a groove along the longitudinal axis.

7. The nail as in claim 1, wherein said body further comprises a internal wall defining a second opening in the body.

8. The nail as in claim 1, wherein said orientation feature provides for the external periphery of the bushing to be substantially spherical.

9. The nail as in claim 1, wherein said body defines a first portion having a first diameter and a second portion having a second diameter, the first diameter being larger than the second diameter.

10. The nail assembly as in claim 9, wherein the first nail opening is located in the first portion.

11. An intramedullary nail assembly for use with a screw in a medullary canal of a long bone, said assembly comprising:
a nail for positioning at least partially in the medullary canal, said nail having a first internal wall defining a nail opening therethrough, said nail further defining a longitudinal axis thereof; ; and
a bushing rotatably positioned at least partially in the nail opening and adapted to receive the screw in a plurality of angular orientations with respect to the longitudinal axis of said nail, said plurality of angular orientations defining a plurality of non-coincident planes.

12. The nail assembly as in claim 11, in which the first mentioned bushing defines a first bushing opening therethrough for receiving at least a portion of said first mentioned screw, and the nail defines a second nail opening therethrough, and in which the assembly includes a second bushing which can be fitted at least partially in the second nail opening and defining a second bushing opening therethrough for receiving at least a portion of a second screw, the first bushing opening and the second bushing opening capable of having different angular orientations with respect to the longitudinal axis of said nail.

13. The nail assembly as in claim 11, wherein said screw includes threads having a first flank, a crest adjacent the first flank and a second flank spaced from the first flank and adjacent the crest, the crest and the first flank forming a first angle therebetween, the crest and the second flank forming a second angle therebetween, the first angle and the second angle being different from each other.

14. The nail assembly as in claim 11, wherein at least a portion of said nail is cannulated along the longitudinal axis.

15. The nail assembly as in claim 11, wherein at least a portion of said nail defines a groove along the longitudinal axis.

16. The nail assembly as in claim 11, wherein at least one of the first mentioned nail opening has a generally oval shape.

17. The nail assembly as in claim 11, wherein the first mentioned bushing has a generally spherical shape.

18. The nail assembly as in claim 11, wherein said nail further comprises a second internal wall defining a second opening.

19. The nail assembly as in claim 11, wherein the external periphery of said nail is substantially cylindrical.

20. The nail assembly as in claim 19, wherein said nail defines a first portion having a first diameter and a second portion having a second diameter, the first diameter being larger than the second diameter.
